# EUROPEAN PATENT SPECIFICATION

(11) **EP 2 296 124 B1**
(45) Date of publication and mention of the grant of the patent: **13.05.2015**
(21) Application number: 09758455.1
(22) Date of filing: 03.06.2009
(51) Int. Cl.: A61B 5/00, G09B 19/16, A61B 5/11, A61B 5/18, G07C 5/08

(54) **SYSTEM FOR AUTOMATIC EVALUATION OF DRIVING BEHAVIOR**
SYSTEM ZUR AUTOMATISCHEN EVALUIERUNG DES FAHRVERHALTENS
SYSTÈME D'ÉVALUATION AUTOMATIQUE DE COMPORTEMENT DE CONDUITE

(30) Priority: 06.06.2008 JP 2008149070
(43) Date of publication of application: 16.03.2011
(73) Proprietor: Yamashiro Driving School, Kyoto 610-0301 (JP); Advanced Telecommunications Research Institute International, Soraku-gun Kyoto 619-0288 (JP)
(72) Inventor: NAKATA, Ryuji, Tsuzuki-gun Kyoto 610-0301 (JP); TAKINO, Akira, Tsuzuki-gun Kyoto 610-0301 (JP); SEGAWA, Makoto, Tsuzuki-gun Kyoto 610-0301 (JP); RENGE, Kazumi, Soraku-gun Kyoto 619-0288 (JP); TADA, Masahiro, Soraku-gun Kyoto 619-0288 (JP); TORIYAMA, Tomoji, Soraku-gun Kyoto 619-0288 (JP); KOGURE, Kiyoshi, Soraku-gun Kyoto 619-0288 (JP)
(74) Representative: Taor, Simon Edward William
(86) International application number: PCT/JP2009/060647
(87) International publication number: WO 2009/148188

(56) References cited:
- DE-B3- 10 256 612
- GB-A- 2 347 573
- JP-A- 2002 211 265
- JP-A- 2002 331 850
- JP-A- 2004 258 956
- JP-A- 2006 227 905
- JP-A- 2007 219 716
- JP-A- 2007 257 333
- JP-A- 2008 046 759
- JP-A- 2008 117 140
- US-A- 5 691 693
- US-A1- 2007 008 151
- US-B1- 6 200 139

## Description

### TECHNICAL FIELD

The present invention relates to a driving action automatic evaluating system. More specifically, the present invention relates to a driving action automatic evaluating system for evaluating a driving action by a driver with respect to a dangerous place.

### BACKGROUND ART

Recently, the number of causalities of traffic accidents is on a declining trend, but a downward trend in the number of occurrences of the accidents itself is not discerned. According to the traffic accident occurring situation statistics (non-patent document 1: National Police Agency Traffic Bureau, traffic accident occurring situation in 2006, February 23, 2007) of National Police Agency, 26.3% of the number of traffic accidents occurred in 2006 is committed by accidents on sudden encounters. In addition, according to the statistics, 52.0% of the accident on sudden encounters occurs at unsignalized intersections in urban areas. Therefore, it may be said that the establishment of technique for prevention of accidents at unsignalized intersections remains a critical issue.

In a conventional traffic accident occurring situation investigation at unsignalized intersections, a right-and-left looking motion by a driver by utilizing a video analysis (including an eye mark recorder) is checked, and vehicle speed at a time of entering an intersection, and operation amounts of an accelerator pedal and/or a brake pedal are measured. Among them, by checking a gaze with an eye mark recorder or a video image, it is possible to know to a certain extent whether a driver can predict a danger at an unsignalized intersection.

Furthermore, in a Patent Document 1 (Japanese Patent Application Laid-Open No. 2002-331850 [B60K 28/06, A61B 3/113, 5/18, B60K 28/16, B60R 21/00]), from a direction frequency distribution of the gaze of the driver detected by an eye mark recorder and operation amounts of an accelerator pedal and/or a brake pedal, a technique of predicting a driving action intention is disclosed.

On the one hand, a taxicab business world takes various measures to reduce accidents by taxi drivers at the present. A reeducation course of driving aimed at taxi drivers who caused an accident is especially frequently performed. However, the reeducation course is just ex post measures, and the effect of the course is reduced with time. It is conceivable that the taxi driver who is considered to have a high probability of causing an accident is preselected, and is made to take the reeducation course, but as a selecting means therefor, it is merely possible to depend on a self-assessed type questionnaire test, such as a driving aptitude test in reality.

However, the eye mark recorder is so extensive, and it is unreasonable to regularly use it during a daily driving. Furthermore, it is considered that in the light of a dangerous drive preventive intention, there is a major difference between a person who moves the foot to the brake pedal before entering the intersection and prepares for emergency (hereinafter to be referred as a person taking a "braking stance") even if the brake pedal is not operated, and a person who does not move the foot from the accelerator pedal, but even if only an operation amount of the accelerator pedal and/or the brake pedal is concentrated, it is impossible to judge whether a "braking stance" is taken.

On the other hand, in the self-assessed type questionnaire test for preselection of a subject to take the reeducation course, the dangerous drive preventive intention of the driver cannot be quantitatively measured, and therefore, it is difficult to make a proper judgment.

Additionally, concern has grown about preventive safety of preventing accidents by motor vehicles before they occur. From the viewpoint of preventing the accident before it occurs, it has become important that not only an abnormal behavior of a motor vehicle is measured or detected, but also the driving characteristics of the driver himself or herself is quantitatively and objectively measured, and if there are needs to be improved, an intention of a safe driving tries to be improved by suitably performing feedback, etc.

At a dangerous place like an accident-prone place, fully decelerating to make a well right and left check by previously predicting a danger, such as "a bicycle sometimes going out from an intersection" is an effective method of preventing accidents (hereinafter, fully deceleration and a right-and-left checking motion at the accident-prone place are generally called an "accident preventive motion"). The strength of the safe driving intention is considered to be estimated by checking whether or not such accident preventive motion is fully performed in daily driving.

US6200139 discloses a system for operator training and evaluation may detect and record information about various actions taken by an operator. Such a system may record what the operator looks at in the course of operating the vehicle to determine whether the operator is looking at the things which the operator should appropriately be checking.

### SUMMARY OF THE INVENTION

Therefore, it is a primary object of the present invention to provide a novel driving action automatic evaluating system.

Another object of the present invention is to provide a driving action automatic evaluating system capable of evaluating whether or not a user takes a necessary accident preventive motion at a dangerous place.

A still another object of the present invention is to provide a driving action automatic evaluating system capable of clearly presenting a driving action by the driver at a dangerous place.

The present invention employs following features in order to solve the above-described problems. It should be noted that reference numerals and the supplements inside the parentheses show one example of a corresponding relationship with the embodiments described later for easy understanding of the present invention, and do not limit the present invention.

According to an aspect of the invention, there is provided a driving action automatic evaluating system as set out in claim 1. Preferred features are set out in claims 2 to 8.

According to another aspect of the invention, there is provided an evaluation program as set out in claim 9.

According to another aspect of the invention, there is provided a driving action evaluating method as set out in claim 10.

The invention is a driving action automatic evaluating system for evaluating a driving action by a driver of a motor vehicle with respect to a predetermined place, comprising: a first movement detector which detects movement data of a head of the driver; a position detector which detects position data of the motor vehicle; a recorder which records at least the movement data of the head detected by the first movement detector and the position data of the motor vehicle detected by the position detector; a motion defining data storager which stores motion defining data defining a minimum accident preventive motion to be performed for each predetermined place by using evaluation items in relation to at least a right-and-left checking motion and a vehicle speed; an evaluator which evaluates whether or not the accident preventive motion is properly performed on the basis of the movement data, the position data and the motion defining data; and an outputter which outputs an evaluation result by the evaluator.

According to the invention, a driving action automatic evaluating system (10) includes a first movement detector (14) and a position detector (20). The first movement detector is for detecting movement data of a head of the driver, and includes an angular velocity sensor, for example. The position detector is for detecting position data of the motor vehicle, and includes a GPS receiver, for example. A recorder (12) records at least the movement data of the head and the position data of the motor vehicle. A motion defining data storager (22) stores motion defining data defining a minimum accident preventive motion to be performed for each predetermined place by using evaluation items in relation to at least a right-and-left checking motion and a vehicle speed. As a predetermined place, a dangerous place may be typically adopted. An evaluator (22, S27, S33, S39, S45, S51, S57) evaluates whether or not the accident preventive motion is properly performed on the basis of the movement data, the position data and the motion defining data. The accident preventive motion is defined by using at least an evaluation item in relation to a right-and-left checking motion and an evaluation item in relation to a vehicle speed, and therefore, by determining whether or not the evaluation item is satisfied, the accident preventive motion by the driver is evaluated. An outputter (22, S79, S83) outputs an evaluation result. As an evaluation result, whether or not the accident preventive motion is properly performed and a point to be improved may be output in a text format.

According to the invention, the minimum accident preventive motions to be performed at a predetermined place are defined by using the evaluation items in relation to the right-and-left checking motion and the vehicle speed, and each of the accident preventive motions is evaluated, and whereby, it is possible to evaluate whether or not the driver takes a necessary accident preventive motion at the predetermined place. Furthermore, an evaluation result of the driving action by the driver with respect to the defined accident preventive motions is output, and therefore, it is possible to clearly present the right and wrong of the driving action by the driver at a predetermined place, the degree of attainment of the accident preventive motion, a point to be improved, and thus, it is possible to make the user of the driving action automatic evaluating system, such as a driver, an instructor of a driving school, etc. easily grasp the driving action by the driver.

According to the invention, there is a driving action automatic evaluating system, wherein the outputter further outputs a waveform indicating a behavior of the head based on at least the movement data of the head.

In the second invention, by an outputter (S73), a waveform indicating a behavior of the head based on at least the movement data of the head is further input, that is, a graph indicating a time variation of the behavior of the head of the driver is further output. Together with the evaluation result of the accident preventive motion at the dangerous place, the graph indicating the behavior of the right-and-left checking motion by the driver is output, and therefore, it is possible to more clearly present how the driving action by the driver is at the dangerous place, and this makes it easy to grasp the driving action by the driver.

According to the invention, there is a driving action automatic evaluating system further comprising a second motion detector which detects movement data of a right toe of the driver, wherein the recorder further records the movement data of the right toe detected by the second motion detector, and the motion defining data storager stores motion defining data defining the accident preventive motion by further using an evaluation item in relation to a position of a right foot.

In the invention, the system further includes a second motion detector (16). The second motion detector is for detecting movement data of a right toe of the driver, and includes an angular velocity sensor similar to the first movement detector. In the motion defining data, the accident preventive motion is defined by using the further evaluation item in relation to the position of the right foot. Accordingly, the evaluation by the evaluator (S65) is performed by further using the evaluation item in relation to the position of the right foot. Thus, it is possible to evaluate whether the accident preventive motion including the condition of the position of the right foot is performed.

A further enhancement is a driving action automatic evaluating system, wherein the outputter further outputs a waveform indicating a behavior of the position of the right foot based on the movement data of the right toe.

By the outputter (S73), a graph indicating a time variation of the behavior of the position of the right foot is output together with the evaluation result of the accident preventive motion including the evaluation of the position of the right foot, and therefore, it is possible to more clearly present the driving action by the driver at the dangerous place including the behavior of the position of the right foot.

A further enhancement is a driving action automatic evaluating system, wherein the motion defining data storager stores data indicating a check direction, the number of checks, a depth of a check, a checking time period, and a checking timing as the evaluation items in relation to the right-and-left checking motion.

It is possible to evaluate the various accident preventive motions defined by using the check direction, the number of checks, the depth of the check, the checking time period, and the checking timing.

A further enhancement is a driving action automatic evaluating system, wherein the outputter indicates an estimated right-and-left checking motion as text information above the waveform indicating the behavior of the head.

By an outputter (S75), a text indicating the estimated right and left checking motion is output above the graph of the waveform indicating the behavior of the head. That is, it is possible to apply a comment of the right-and-left checking motion to the graph of the waveform indicating the behavior of the head. Accordingly, it is possible to easily grasp the right-and-left checking motion by the driver.

A further enhancement is a driving action automatic evaluating system, wherein the outputter further outputs a waveform indicating a vehicle speed based on the position data.

By an outputter (S73), a waveform of a vehicle speed, that is, a graph indicating a time variation of the speed of the motor vehicle can be output. Accordingly, the time variation of the head behavior can be viewed while the time variation of the vehicle speed is viewed, and this makes it easy to grasp the right-and-left checking motion by the driver.

A further enhancement is a driving action automatic evaluating system, wherein the outputter outputs text information indicating a situation of the motor vehicle estimated based on the position data above the waveform.

By an outputter (S77), a text indicating an estimated result of a situation of the motor vehicle can be output above the graph. Accordingly, a situation of the motor vehicle at a time when the motor vehicle enters the dangerous place can be presented above the graph of the waveform of the right-and-left checking motion, the waveform of the vehicle speed, etc., and therefore, it becomes possible to more easily grasp the driving action by the driver.

A further enhancement is a driving action automatic evaluating system further comprising a scorer which gives a score on the basis of an evaluation by the evaluator, wherein the scorer gives 0 point to the driver when the vehicle speed does not satisfy the motion defining data, or when no right-and-left checking motion is performed before passing by the predetermined place.

By a scorer (22, S85-S101), a score based on the evaluation of the accident preventive motion is given, and 0 point is given when the condition defining the vehicle speed is not satisfied, or when no right-and-left checking motion is performed at the predetermined place at all. Thus, in a case that deceleration is not performed to a speed at which a right and left check can be performed, and in a case that the driver lacks the accident preventive intention, the driver is not given a pass mark.

A further aspect is an evaluation program stored in a storage medium readable by a computer by a driving action automatic evaluating system for evaluating a driving action of a driver of a motor vehicle with respect to a predetermined place and causing the computer to function as: a first movement detector which detects movement data of a head of the driver; a position detector which detects position data of the motor vehicle; a recorder which records at least the movement data of the head detected by the first movement detector and the position data of the motor vehicle detected by the position detector; a motion defining data storager which stores motion defining data defining a minimum accident preventive motion to be performed for each predetermined place by using evaluation items in relation to at least a right-and-left checking motion and a vehicle speed; an evaluator which evaluates whether or not the accident preventive motion is properly performed on the basis of the movement data, the position data and the motion defining data; and an outputter which outputs an evaluation result by the evaluator.

It is possible to expect an advantage similar to the first invention.

A further enhancement is an evaluation program causing the computer to further function as a second motion detector which detects movement data of a right toe of the driver, wherein the recorder further records the movement data of the right toe detected by the second motion detector, and the motion defining data storager stores motion defining data defining an accident preventive motion by using a further evaluation item in relation to a position of a right foot.

It is possible to expect an advantage similar to the third invention.

A further enhancement is an evaluation program stored in a storage medium readable by a computer of a driving action automatic evaluating system for evaluating a driving action by a driver of a motor vehicle with respect to a predetermined place, and causing the computer to execute: a first movement detecting step for detecting movement data of a head of the driver; a position detecting step for detecting position data of the motor vehicle; a recording step for recording at least the movement data of the head detected by the first movement detector and the position data of the motor vehicle detected by the position detector; a motion defining data storing step for storing motion defining data defining a minimum accident preventive motion to be performed for each predetermined place by using evaluation items in relation to at least a right-and-left checking motion and a vehicle speed; an evaluating step for evaluating whether or not the accident preventive motion is properly performed on the basis of the movement data, the position data and the motion defining data; and an outputting step for outputting an evaluation result by the evaluating step.

It is possible to expect an advantage similar to the first invention.

A further enhancement is an evaluation program causing a computer to execute a second motion detecting step for detecting movement data of a right toe of the driver, wherein the recording step further records the movement data of the right toe detected by the second motion detector, and the motion defining data storing step stores motion defining data defining the accident preventive motion by using a further evaluation item in relation to a position of a right foot.

A further aspect is an driving action evaluating method for evaluating a driving action of a driver of a motor vehicle with respect to a predetermined place, and causing a computer to execute: a first movement detecting step for detecting movement data of a head of the driver; a position detecting step for detecting position data of the motor vehicle; a recording step for recording at least the movement data of the head detected by the first movement detector and the position data of the motor vehicle detected by the position detector; a motion defining data storing step for storing motion defining data defining a minimum accident preventive motion to be performed for each predetermined place by using evaluation items in relation to at least a right-and-left checking motion and a vehicle speed; an evaluating step for evaluating whether or not the accident preventive motion is properly performed on the basis of the movement data, the position data and the motion defining data; and an outputting step for outputting an evaluation result by the evaluating step.

It is possible to expect an advantage similar to the first invention.

A further enhancement is a driving action evaluating method further including a second motion detecting step for detecting movement data of a right toe of the driver, wherein the recording step further records the movement data of the right toe detected by the second motion detector, and the motion defining data storing step stores motion defining data defining the accident preventive motion by using a further evaluation item in relation to a position of a right foot.

It is possible to expect an advantage similar to the first invention.

According to the present invention, whether or not a minimum accident preventive motion to be performed for each predetermined place that is defined by using evaluation items in relation to at least a right-and-left checking motion and a vehicle speed is properly performed on the basis of the movement data of the head and the position data of the motor vehicle, and therefore, it is possible to evaluate whether or not the driver performs a necessary accident preventive motion at a predetermined place, such as a dangerous place, etc. Furthermore, an evaluation result of the driving action by the driver with respect to the defined accident preventive motions is output, and therefore, it is possible to clearly present the right and wrong of the driving action by the driver at a predetermined place, such as a dangerous place, the degree of attainment of the accident preventive motion, a point to be improved, etc, and it is possible to easily grasp the driving action by the driver.

The above described objects and other objects, features, aspects and advantages of the present invention will become more apparent from the following detailed description of the present invention when taken in conjunction with the accompanying drawings.

### BRIEF DESCRIPTION OF THE DRAWINGS

Figure 1 is a block diagram showing a configuration of a driving action automatic evaluating system of one embodiment of the present invention.
Figure 2 is an illustrative view showing one example of an angular velocity sensor attached to a cap of a driver.
Figure 3 is an illustrative view showing one example of an angular velocity sensor attached to a right toe of the driver.
Figure 4 is an illustrative view showing a situation in which the right toe of the driver is moved from an accelerator pedal to a brake pedal.
Figure 5 is an illustrative view showing one example of dangerous place information.
Figure 6 is a flowchart showing a part of one example of an operation of the driving action automatic evaluating system.
Figure 7 is a flowchart showing a part of a sequel to Figure 6.
Figure 8 is a flowchart showing a part of a sequel to Figure 7.
Figure 9 is a flowchart showing a part of a sequel to Figure 7 and Figure 8.
Figure 10 is a flowchart showing a part of a sequel to Figure 9.
Figure 11 is a flowchart showing a sequel to Figure 10.
Figure 12 is an illustrative view showing one example of an evaluation result displaying screen.

### BEST MODE FOR PRACTICING THE INVENTION

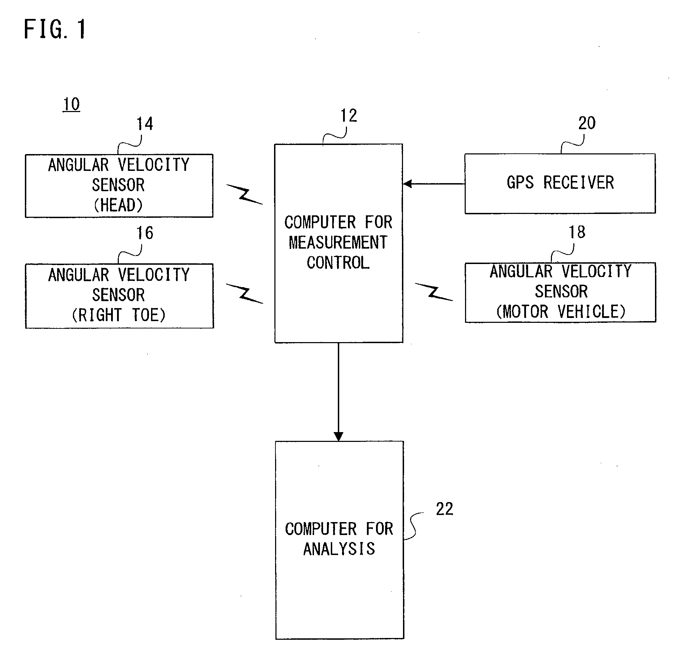

Referring to Figure 1, a driving action automatic evaluating system (hereinafter simply referred to as "system") 10 of this embodiment is for evaluating a driving action by a driver of a motor vehicle with respect to a dangerous place, and includes a measuring unit (measuring device) and an analyzing unit (analyzing device). More specifically, the system 10 includes a computer for measurement control 12, angular velocity sensors 14, 16 and 18, and a GPS receiver 20 as a measuring unit, and a computer for analysis 22 as an analyzing unit.

Here, in this embodiment, separate computers are used as a computer for measurement control 12 and a computer for analysis 22, but one computer may have both of the functions.

The computer for measurement control 12 is for measuring data. As a computer for measurement control 12, a PDA (personal digital assistance) is used in this embodiment, but in another embodiment, a personal computer, a computer of an electronic control unit of a motor vehicle, a computer of a car navigation system, etc. may be used.

The computer for measurement control 12 is, although illustration is omitted, provided with a CPU, a ROM, a RAM, a communication device, etc. The ROM previously stores a control program and necessary data. The RAM is used as a work memory and a buffer memory, and temporarily stores generated data and acquired data, etc. The communication device transmits and receives data with devices such as angular velocity sensors 14, 16, 18, etc. to be connected to this computer for measurement control 12.

In this embodiment, the angular velocity sensor 14 is applied as a movement detector for detecting a movement of a head of the driver, and the angular velocity sensor 16 is applied as a movement detector for detecting a movement of a right toe of the driver. As described later, the angular velocity sensor 14 is for detecting a right and left visually checking motion (right-and-left checking motion) by the driver, and the angular velocity sensor 16 is for detecting a braking stance of the driver, and therefore, as angular velocity sensors 14 and 16, one capable of detecting at least one-axis angular velocity may be used. Accordingly, a one-axis angular velocity sensor, a two-axis angular velocity sensor or a three-axis angular velocity sensor can be used. Furthermore, as angular velocity sensors 14 and 16, the same kind of sensors may be used. Each of the angular velocity sensors 14, 16 detects an angular velocity about a predetermined axis at a predetermined cycle (at 100 Hz, for example) (every predetermined time). Furthermore, each of the angular velocity sensors 14, 16 has a communication function, and transmits the detected angular velocity data to the computer for measurement control 12 for each predetermined time interval or at a predetermined timing. In addition, in this embodiment, each of the angular velocity sensors 14, 16 transmits and receives data with the computer for measurement control 12 by a short distance wireless communication, such as a Bluetooth (registered trademark). Here, in another embodiment, each of the angular velocity sensors 14, 16 may be connected to the computer for measurement control 12 by wire, but may be desirably connected in a wireless manner so as not to prevent the driver from driving the motor vehicle.

The angular velocity sensor 14 is attached to the head of the driver in order to detect a visually checking motion by the driver. When the driver performs a right and left visually checking motion, not only the eyeballs but also the head are generally moved more or less. Thus, by measuring the movement of the head, changes in a gazing direction can be estimated without placing a significant burden on the driver.

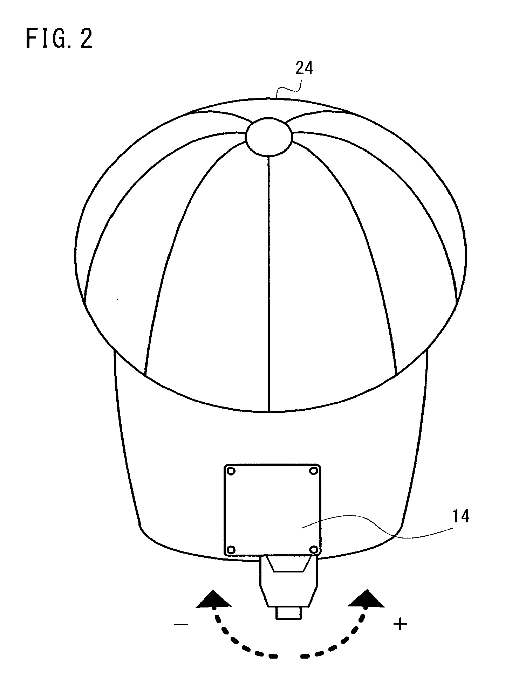

Additionally, for the purpose of implementing easy attachment to the head, a cap 24 is used as shown in Figure 2 in this embodiment, and the angular velocity sensor 14 is attached to a brim of the cap 24, for example. Figure 2 shows a situation that the cap 24 is viewed from above. The angular velocity sensor 14 is attached to the cap 24 so as to detect an angular velocity in correspondence with a rotation about at least an axis of a vertical direction. If the driver puts on the cap 24 attached with the angular velocity sensor 14, angular velocity data in correspondence with a rotation (turn) of the head of the driver in a horizontal direction can be measured, that is, a rotation of the head of the driver, in other words, a visually checking motion can be detected and measured. As to a detecting direction of the angular velocity about the axis in the vertical direction of the angular velocity sensor 14, a counterclockwise rotation is set to a positive, and a clockwise rotation is set to a negative direction in Figure 2. If the driver who puts on the cap 24 turns to the left, positive angular velocity data is detected, and if the driver turns to the right, the negative angular velocity data is detected. Thus, by analyzing the speed data of the angular velocity sensor 14, the computer for measurement control 12 judges whether or not the driver turns his or her head right or left, that is, whether or not a light or left visually checking motion is performed.

Here, the angular velocity sensor 14 may be attached to the head by other methods, such as a hair band, a hair pin, a bandanna, a head band, etc. In addition, except for direct attachment of the angular velocity sensor 14 to the head, it may be attached by using means, such as pierced earrings, earrings, glasses, a mask, a nose ring, etc.

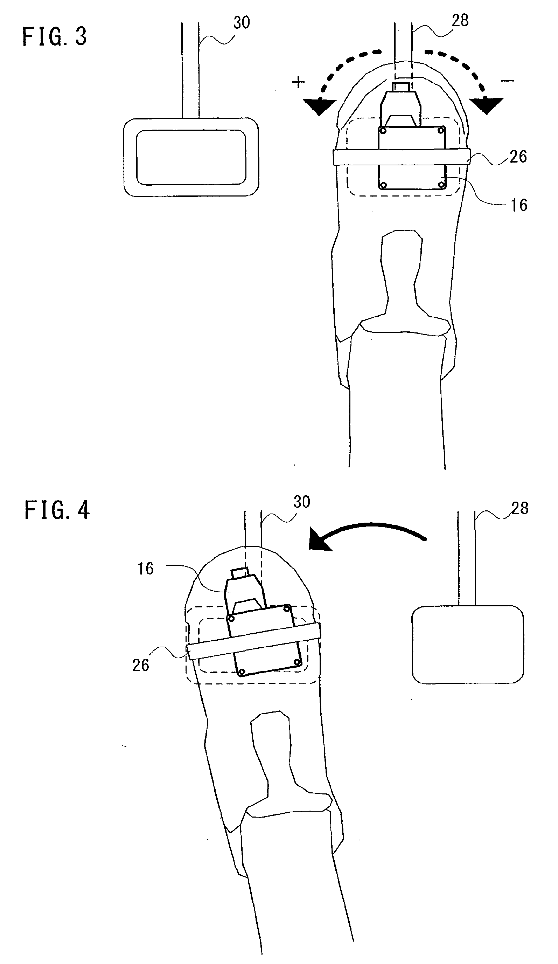

The angular velocity sensor 16 is attached to the right toe of the driver for detecting a braking stance of the driver as shown in Figure 3. The braking stance is considered to be an index to know whether or not the driver predicts a danger, whether or not a preparation in a case of an emergency is made, and therefore, by attaching the angular velocity sensor 16 to the right toe, the dangerous drive preventive intention is measurable.

The angular velocity sensor 16 is attached to the right toe of the driver by using a band 26, etc. Here, the angular velocity sensor 16 may be attached to the right toe by other methods, and the angular velocity sensor 16 may be contained at a part of a toe of driving shoes. In addition, it is conceivable that this may be put on the foot by using means, such as an anklet (leg ring put on the anklet), socks, nail (artificial nail), etc.

In addition, Figure 3 shows the angular velocity sensor 16 when viewed from the driver. The angular velocity sensor 16 is attached to the right toe of the driver so as to detect an angular velocity in correspondence with a rotation about at least an axis of a vertical direction. As to a detecting direction of the angular velocity about the axis in the vertical direction of the angular velocity sensor 16, a counterclockwise rotation is set to a positive, and a clockwise rotation is set to a negative direction in Figure 3.

During driving of the motor vehicle, the right foot of the driver is put on an accelerator pedal 28 as shown in Figure 3. In a case that a braking stance is taken or deceleration is made, the right foot of the driver is moved to a side of a brake pedal 30 as shown in Figure 4, and in a case that the speed is maintained or acceleration is made thereafter, the right foot is returned to a side of the accelerator pedal 28 as shown in Figure 3. The movement between the accelerator pedal 28 and the brake pedal 30 causes a rotation in a horizontal direction, and therefore, by detecting a rotation of the right foot by the angular velocity sensor 16, it is possible to judge whether the driver takes a braking stance.

Returning to Figure 1, the angular velocity sensor 18 is for detecting a behavior of a motor vehicle (car). As an angular velocity sensor 18, an angular velocity sensor similar to the above-described angular velocity sensors 14 and 16 is used. In this embodiment, the angular velocity sensor 18 is attached to a predetermined position on a dashboard by a fixing member such as an adhesive tape, etc. The angular velocity sensor 18 is attached so as to detect an angular velocity in accordance with a rotation about at least an axis of a vertical direction. Accordingly, angular velocity data in correspondence with a rotation (turn) of the motor vehicle in a horizontal direction can be measured, that is, a rotation, in other words, making a right turn or a left turn of the motor vehicle can be detected and measured. As to a detecting direction of the angular velocity about the axis in the vertical direction of the angular velocity sensor 18, similar to the angular velocity sensors 14 and 16, the counterclockwise rotation is set to a positive, and the clockwise rotation is set to a negative direction. If the motor vehicle makes a left turn, positive angular velocity data is detected, and if it makes a right turn, negative angular velocity data is detected. Thus, by analyzing the speed data of the angular velocity sensor 18, the computer for measurement control 12 judges whether or not the motor vehicle makes a right turn or a left turn.

The GPS receiver 20 is a position detecting device for detecting a current position of the motor vehicle, and is attached to a roof of the motor vehicle, for example. The GPS receiver receives a signal from a GPS satellite at a predetermined cycle (at 1 Hz, for example) (every predetermined time) to calculate coordinates (latitude, longitude, altitude) at the current position, and outputs position data including the coordinates of the current position to the computer for measurement control 12.

Here, the system 10 may use an acceleration sensor, a speed sensor, the angular velocity sensor 18, etc. mounted on the motor vehicle for estimating a position in a place where a signal from the GPS satellite is not received. Furthermore, the GPS receiver 20 may use a GPS receiver of a car navigation system mounted on the motor vehicle.

The computer for measurement control 12 records angular velocity data received from the angular velocity sensors 14, 16 and 18 and position data received from the GPS receiver 20 as measured data in the memory. Here, as to the angular velocity data and the position data, an angular velocity value and coordinates are stored by being brought into correspondence with a detection time or an acquisition time, respectively.

The measured data recorded while the motor vehicle is moving is transmitted from the computer for measurement control 12 to the computer for analysis 22 so as to be fetched by the computer for analysis 22. Here, the measured data may be fetched by the computer for analysis 22 via a storage medium. The computer for analysis 22 is for evaluating a driving action by a driver by analyzing the measured data, and outputting an evaluation result. As a computer for analysis 22, a personal computer is used in this embodiment. The computer for analysis 22, although the illustration is omitted, is provided with a CPU, a ROM, a RAM, an HDD, a communication device, an input device, etc., and is further provided with an output device (display or printer, etc.) for outputting an evaluation result. The ROM previously stores a control program (including an analysis program) and necessary data. The RAM is used as a work memory and a buffer memory, and temporarily stores generated data, acquired data, etc. The communication device transmits and receives data with the computer for measurement control 12.

In order to automatically evaluate a driving action, it is first necessary to define a reference of "full marks". A method of regarding a model driving by an instructor as full marks, and giving a score based on a difference therefrom is conceivable, but in this case, it is impossible to perform evaluation at a place where there is no model driving data. Thus, in this embodiment, a method of defining minimum safety checking/ ensuring motions (accident preventive motions) to be done at a certain place, and evaluating a driving action at this place depending on the degree of attainment with respect to them is adopted. That is, a minimum driving capable of previously avoiding a danger rather than an ideal driving is taken as full marks.

As a result of performing an interview study on an instructor of a driving school, a finding that the instructors perform driving evaluations by focusing on the following points can be acquired. That is, as to a right-and-left checking motion, there are (1) a check direction (right and left), (2) the number of checks, (3) a depth of a check (degree of check with the mirror, deeper visual check, looking-back check (checking the left side at a time of left turn)), (4) a duration of check, that is, checking time period (carless look or careful look), (5) a timing when a check is performed, that is, checking timing (before entering the dangerous place, immediately before entering the dangerous place, during entering the dangerous place), (6) a speed of the motor vehicle at a dangerous place, and (7) which pedal the right foot has to be put on (braking stance is performed).

On the basis of this finding, in this embodiment, for each dangerous place, a minimum accident preventive motion to be done at this place is defined by using the aforementioned evaluation items from (1) to (7), and according to the degree of attainment of these items, the evaluation is performed. Here, a definition of the accident preventive motion is performed by an instructor working at a driving school for 25 years. At a driving action evaluation, only when all the evaluation items (1)-(7) are satisfied, it is regarded that the accident preventive motion is properly performed.

The computer for analysis 22 previously stores information in relation to dangerous places along a course on which a driver is made to drive. Each of the dangerous places is a typically accident-prone place. In a case that the predetermined course is set to a public road, a course for evaluating a driving action is set so as to include dangerous places on the public road. Furthermore, the course for evaluating the driving action may be provided in a driving school and an establishment, etc., and in this case, by reproducing the shape of the accident-prone place along the course, the dangerous place is provided.

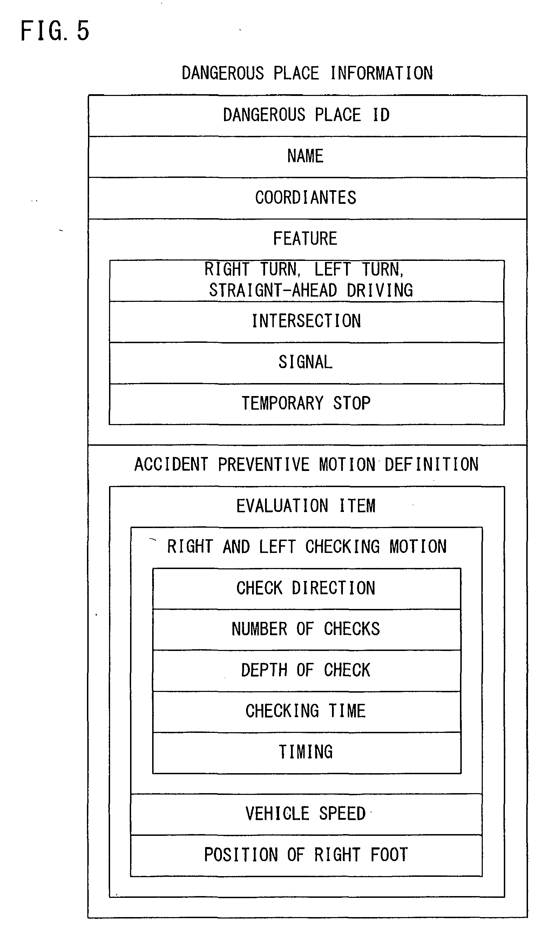

Figure 5 shows one example of dangerous place information. Together with various information in relation to the dangerous place, the definition data of an accident preventive motion set to the dangerous place is stored by being brought into correspondence with identifying information (dangerous place ID) of the dangerous place.

In this embodiment, the information of the dangerous place includes a name, coordinates, a feature, etc. The name is a name set to a dangerous place. For example, in a case that the dangerous place is an intersection that requires a left turn from an old road 3 to an old road 4, a designation of "old road 3→old road 4 and left turn" is set. The coordinates indicate a position of the dangerous place, and is represented by a latitude, a longitude and an altitude similar to the position data of the motor vehicle detected by the GPS receiver 20. Furthermore, the dangerous place is actually not one point but a region with an area or a width, and therefore, coordinates data indicating the region is stored. The feature indicates a feature in relation to driving at the dangerous place, and stores which way the motor vehicle passes by the dangerous place, right turn, left turn or straight-ahead driving, whether or not the dangerous place is an intersection, whether or not a signal is set to the dangerous place, whether or not a temporary stop is obliged at the dangerous place, for example.

Additionally, definition data of the accident preventive motion set to the dangerous place is stored. In the dangerous place, one or a plurality of accident preventive motions are set. As evaluation items, (1) a check direction, (2) the number of checks, (3) a depth of a check, (4) a checking time period, (5) a checking timing of a right-and-left checking motion, and (6) a vehicle speed and (7) the information of a right foot position are stored.

For example, in a case of checking on the left side before making left turn, the left as (1) a check direction, once as (2) the number of checks, a looking-back check in a left rear direction as (3) a depth of the check, no good of a carless look as (4) a checking time period, an immediate before making a left turn as (5) a checking timing, 20 km/h or lower as (6) a vehicle speed, and no definition as (7) a position of the right foot are defined as an accident preventive motion. In an item for which no evaluation is performed, data indicating that definition is not made (no definition) is set.

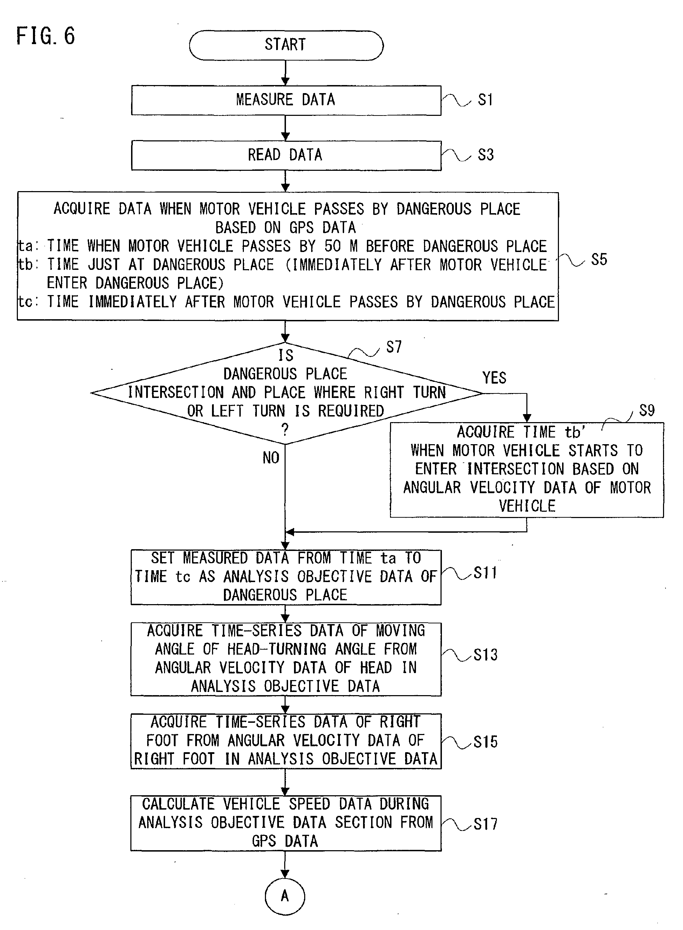

Successively, with reference to flowcharts shown from Figure 6 to Figure 11, one example of a specific operation of the system 10 is explained.

First, in a step S1, data measurement is performed by the computer for measurement control 12. The data measurement is performed by making the driver attached with the angular velocity sensors 14 and 16 drive along the predetermined course. The angular velocity data of the angular velocity sensor 14 of the head, the angular velocity data of the angular velocity sensor 16 of the right toe and the angular velocity data of the angular velocity sensor 18 of the motor vehicle are sampled at 100 Hz, for example, and the position data of the GPS receiver 20 is sampled at 1 Hz, for example, and each of data is recorded in the computer for measurement control 12.

Next, in a step S3, the measured data is fetched in the computer for analysis 22. The fetching of the data is performed by transmitting the measured data from the computer for measurement control 12 to the computer for analysis 22 after driving, or the measured data is stored in a storage medium such as a memory card, and the measured data is read from the storage medium into the computer for analysis 22. Processing after a succeeding step S5 is executed by the computer for analysis 22. The processing after the step S5 is executed for each dangerous place.

In the step S5, a time when the motor vehicle passes by the dangerous place is acquired on the basis of the GPS data. More specifically, on the basis of latitude information, longitude information, coordinates data of the dangerous place of the GPS data, the time when the motor vehicle enters in the dangerous place previously set is acquired. At this time, in this embodiment, three kind of times, such as a time "ta" when the motor vehicle passes by 50 m before the dangerous place, a time "tb" just at the dangerous place (immediate after entering), and a time "tc" immediately after the motor vehicle passes by the dangerous place are acquired. Here, the time "ta" is for deciding a starting position of analysis objective data for evaluating a driving action at a dangerous place, and the place 50 m before the dangerous place is regarded as a starting point in this embodiment, but the predetermined distance may be arbitrarily changed.

Additionally, in a step S7, it is determined whether or not the dangerous place is an intersection, and a right turn or a left turn has to be made on the basis of the dangerous place information. If "YES" in the step S7, the time "tb"' when the motor vehicle starts to enter the intersection is acquired on the basis of the angular velocity data of the motor vehicle in a step S9. That is, the time when the motor vehicle starts to make a right turn or a left turn is estimated. More specifically, from the time "ta" when the motor vehicle passes by 50 m before the dangerous place acquired from the GPS, the output of the angular velocity sensor is started to be integrated, and the time when the integral value is above a predetermined threshold value (5 degrees in this embodiment) is regarded as a time tb' when the motor vehicle starts to enter the intersection. Here, in this embodiment, the sampling rate of the angular velocity sensor 18 is 100 Hz, and this is denser than that of the GPS receiver 20, and thus, the time "tb"' when the motor vehicle starts to enter the intersection estimated by integrating the output from the angular velocity sensor is given a high priority to the time "tb" just at the dangerous place (immediate after entering) estimated from the GPS data. On the other hand, if "NO" in the step S7, the process proceeds to a step S 11 as it is.

In the step S11, the measured data from the time "ta" to the time "tc" is set as the analysis objective data of the dangerous place.

Successively, in a step S 13, time-series data of the head-turning angle is acquired from the angular velocity data of the head in the analysis objective data. That is, the output from the angular velocity sensor of the head is integrated, and the head-turning angle at a certain time is estimated. Here, it is known that merely integrating the angular velocity data causes accumulated errors, resulting in an estimated value greatly different from an actual value. In order to deal with this, a next method is adopted in this embodiment. While the motor vehicle is moving, that is, when the speed of the motor vehicle is not 0 km/h, the head of the driver is considered to face front. Thereupon, if the angular velocity more than ωhead is not detected for a time thead during the moving, the head-turning angle is reset to 0. The parameter for deciding the reset of the cumulative error is empirically evaluated, and in this embodiment, the time thead is 3.0 seconds, and the ωhead is 30. 0 deg (degrees)/s.

Furthermore, in a step S 15, time-series data of the moving angle of the right foot is acquired from the angular velocity data of the right foot in the analysis objective data. That is, the output from the angular velocity sensor of the right toe is integrated to estimate the position of the right foot at a certain time. Similar to the above-described head-turning angle, in order to eliminate the errors in the integration, a following method is adopted. From the finding by a study by the video, during moving, the right foot of the driver is basically put on the accelerator pedal 28, and is often moved to the brake pedal 30 as necessary. Thereupon, in a case that the angular velocity equal to or more than ωtoe is not detected for the time ttoe during moving, the position of the foot is reset to the position of the accelerator pedal. In this embodiment, the time ttoe empirically evaluated is 15.0 seconds, and the ωtoe is 30.0 deg/s.

Furthermore, in a step S17, vehicle speed data during the analysis objective data section is calculated from the GPS data. The vehicle speed is calculated from the moving distance for one second, for example.

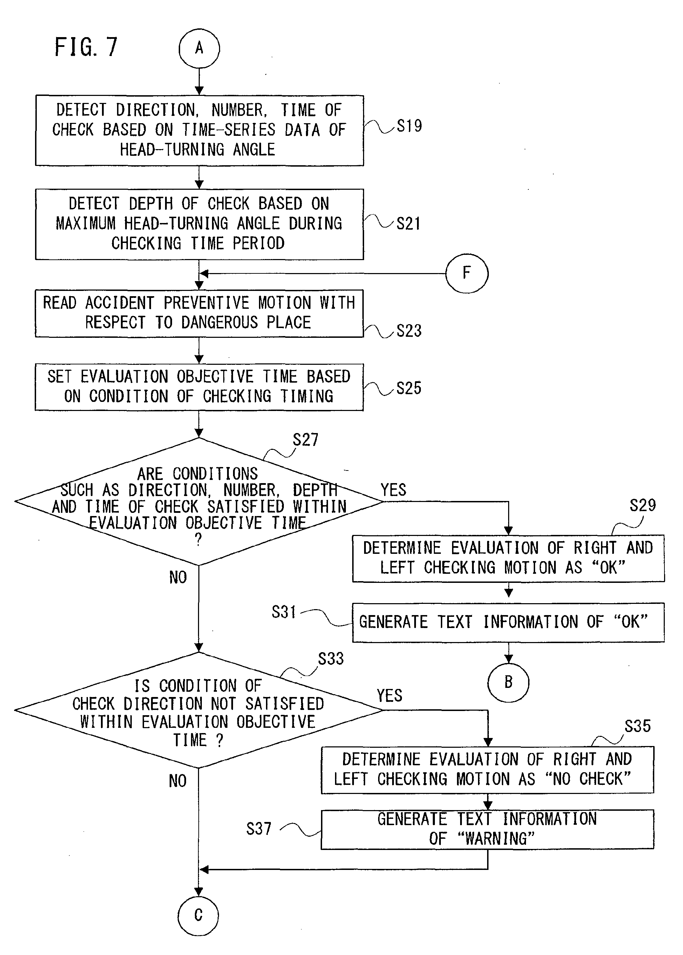

Successively, in a step S 19 in Figure 7, the direction of the right-and-left check, the number of checks and the checking time period are detected on the basis of the time-series data of the head-turning angle. More specifically, in a case that an angle equal to or more than the absolute value of 15 degrees occurs in the time-series data of the head-turning angle, it is regarded that a right and left checking motion is performed. That is, the number of checks of the evaluation item (2) becomes +1. Furthermore, while the angle equal to or more than 15 degrees continuously occurs thereafter, it is regarded that the one right and left checking motion continues. The continued time is regarded as a checking time period of the evaluation item (5). In addition, as shown in Figure 2, in a case that the sign of the head-turning angle is positive, the check direction of the evaluation item (1) is the left direction, and in a case of the negative direction, the check direction of the evaluation item (1) is the right direction.

In addition, in a step S21, a maximum head-turning angle is calculated during the checking time period, and the depth of the right and left check of the evaluation item (3) is detected on the basis of the magnitude. In this embodiment, the depth of the check is evaluated into three levels. More specifically, if the maximum head-turning angle is equal to or more than 15 degrees and less than 35 degrees, a shallow checking motion is evaluated, if the maximum head-turning angle is equal to or more than 35 degrees and less than 55 degrees, a normal checking motion is evaluated, and if the maximum head-turning angle is equal to or more than 55 degrees, a looking-back checking motion is evaluated. The threshold values for classifying these check motions were acquired from the measured data by the experiments and the video analysis.

Successively, in a step S23, the definition data of the accident preventive motion set to the dangerous place is read from the ROM or the HDD to the memory (RAM). A plurality of accident preventive motions can be set to the dangerous place, and whether or not the motion is properly performed is checked for each accident preventive motion.

First, in a step S25, an evaluation objective time is set on the basis of a condition of a checking timing of the right-and-left checking motion. For example, if the checking timing is set to the "immediate before making a left turn", only the time from a predetermined time before the time "tb"' when the motor vehicle starts to enter the intersection (3 seconds in this embodiment) to the time "tb"' when the motor vehicle starts to enter the intersection shall be an object to be evaluated..

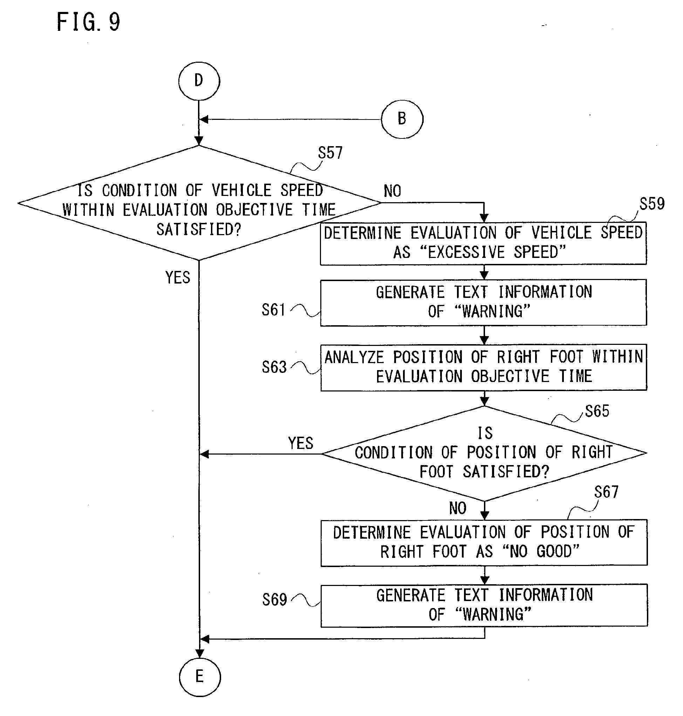

Then, in a step S27, it is determined whether or not the conditions, such as the direction, the number, the depth and the time of a check are satisfied within the evaluation objective time. If "YES" in the step S27, that is, if the evaluation items (1) to (5) of the right-and-left checking motion are satisfied, the evaluation of the right-and-left checking motion is determined as "OK" in a step S29, and data indicating the determination result is stored. Furthermore, in a step S31, the text information of "OK" as to the right-and-left checking motion is generated in the memory. The text information is for displaying an evaluation result of the right-and-left checking motion on the evaluation result screen. Here, the text information for displaying that the right-and-left checking motion is properly performed is generated. When the processing in the step S31 is completed, that is, if the evaluation of the right-and-left checking motion is completed, the process proceeds to a step S57 in Figure 9.

On the other hand, if "NO" in the step S27, that is, if the right-and-left checking motion is not properly performed within the evaluation objective time, the degree of attainment is further checked. That is, in a step S33, it is determined whether or not the condition of the check direction is not satisfied within the evaluation objective time. If "YES" in the step S33, that is, if a direction check motion toward the direction to be checked is not performed, the evaluation of the right-and-left checking motion is determined to be a "no check" in a step S35, and data indicating the determination result is stored in the memory. Furthermore, in a step S37, as to the right-and-left checking motion, text information of "warning" is generated in the memory. The right-and-left checking motion is not performed, and therefore, the text information for warning the matter is generated. If the processing in the step S37 is completed, or if "NO" in the step S33, the process proceeds to a step S39.

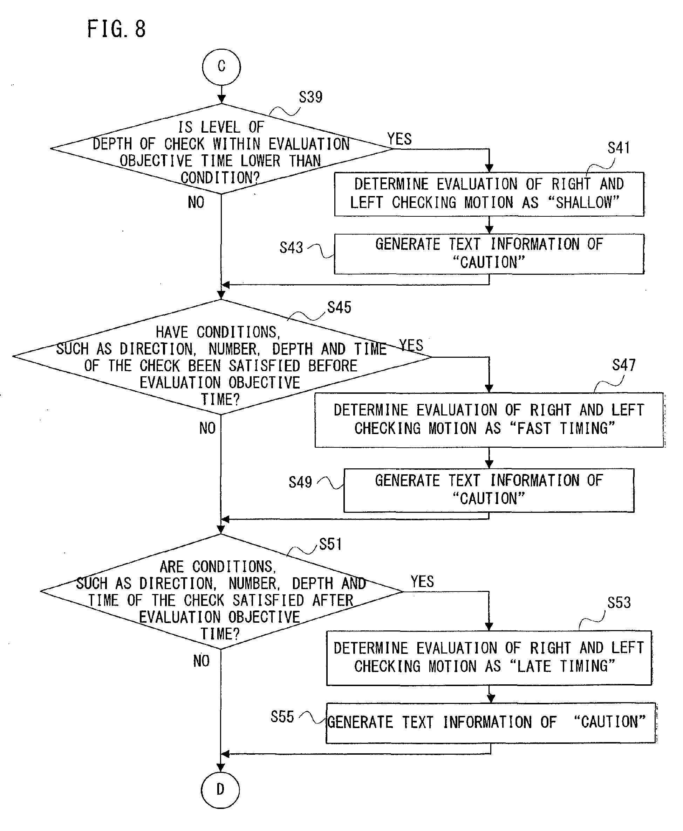

In the step S39 in Figure 8, it is determined whether or not the level of the depth of the check within the evaluation objective time is lower than the condition. That is, if there is a check operation in a check direction within the evaluation objective time, but the maximum head-turning angle does not satisfy the condition, the evaluation of the right-and-left checking motion is determined to be "shallow" in a step S41, and data indicating the determination result is stored in the memory. Furthermore, in a step S43, the text information of "caution" as to the right-and-left checking motion is generated in the memory. Since the checking motion shallower than the condition is performed, text information for cautioning the matter is generated. When the processing in the step S43 is completed, or if "NO" in the step S39, the process proceeds to a step S45.

In the step S45, it is determined whether or not the conditions, such as the direction, the number, the depth and the time of a check have been satisfied before the evaluation objective time. If "YES" in the step S45, that is, if it is determined that a right-and-left checking motion has been performed during a predetermined time before the evaluation objective time, the evaluation of the right-and-left checking motion is determined to be a "fast timing" in a step S47, and the data indicating determination result is stored in the memory. Furthermore, in a step S49, the text information of "caution" as to the right-and-left checking motion is generated in the memory. Since a checking motion at a timing faster than the condition is performed, the text information for cautioning the matter is generated. When the step S49 is completed, or if "NO" in the step S45, the process proceeds to a step S51.

In the step S51, it is determined whether or not the conditions, such as the direction, the number, the depth and the time of a check are satisfied after the evaluation objective time. If "YES" in the step S51, that is, if it is determined that a right-and-left checking motion is performed during a predetermined time immediately after the evaluation objective time, the evaluation of the right-and-left checking motion is determined to be a "late timing" in a step S53, and the data indicating determination result is stored in the memory. Furthermore, in a step S55, the text information of "caution" as to the right-and-left checking motion is generated in the memory. Since a checking motion at a timing later than the condition is performed, the text information for cautioning the matter is generated. When the step S55 is completed, or if "NO" in the step S51, the process proceeds to the step S57.

In the step S57 in Figure 9, it is determined whether or not the condition of the vehicle speed within the evaluation objective time is satisfied. In a case that "NO" in the step S57, that is, if a vehicle speed above the condition is detected with reference to the vehicle speed data, the evaluation of the vehicle speed is determined to be an "excessive speed" in a step S59, and data indicating the determination result is stored in the memory. Furthermore, in a step S61, text information of "warning" as to the vehicle speed is generated in the memory. Since the vehicle speed is above the speed required for a safety check and a safety ensuring, the text information for warning this matter is generated.

Here, in a case that the dangerous place is a place where a temporary stop is required, and the accident preventive motion is a temporary stop, the condition of the vehicle speed is set to 0 km/h, and it is determined whether or not the vehicle speed becomes 0 km/h within the evaluation objective time. Then, if it is not satisfied, the evaluation of the vehicle speed is determined to be "failure of the temporary stop", and text information for alarming the failure of the temporary stop is generated.

Successively, in a step S63, the position of the right foot within the evaluation objective time is analyzed on the basis of the time-series data of the moving angle of the right foot. Then, in a step S65, it is determined whether or not the condition of the position of the right foot is satisfied. In a case that "NO" in the step S65, that is, in a case that the position of the right foot is determined to be a position opposite to the condition, the evaluation of the position of the right foot is determined to be "no good" in a step S67, and data indicating the determination result is stored in the memory. Furthermore, in a step S69, text information of "warning" as to the position of the right foot is generated in the memory. Since the right foot is at a position different from the condition, the text information warning this matter is generated. When the step S69 is completed, if "YES" in the step S65, or if "YES" in the step S57, the process proceeds to a step S71.

In this embodiment, in a case that the condition of the vehicle speed is not satisfied, the condition of the position of the right foot is determined. This is basically to confirm whether or not a braking stance is taken so as to quickly decelerate in a case that the vehicle speed is not fully decelerated. That is, this follows the philosophy that in a case that the vehicle speed is fully decelerated, there is no harm in not taking a braking stance in view of the safety ensuring. In a case that dangerous place is an intersection, and a right turn or a left turn is required, the speed is required to be fully decelerated for safety confirmation and safety ensuring, and thus, it is conceivable that by setting a proper value to the condition of the vehicle speed, the safety is ensured. On the other hand, if the dangerous place is a place where the motor vehicle goes straight ahead, the speed can be set to a value higher than that when a right turn or a left turn is required, and it is conceivable that a brake position is set as a condition of the position of the right foot, and the degree of safety is heightened by a braking stance. It should be noted that in another embodiment, the position of the right foot may be determined irrespective of the vehicle speed, and in this case, the processing from the step S63 to the step S69 may be successively executed if "YES" is determined in the step S57.

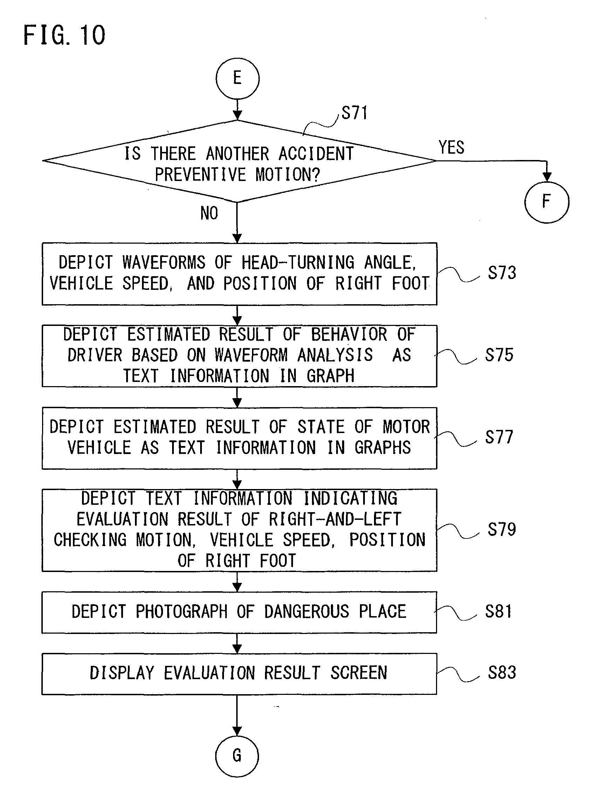

In the step S71 in Figure 10, it is determined whether or not there is another accident preventive motion. If "YES" in the step S71, that is, if another accident preventive motion is set to the dangerous place, the process returns to the step S23 in Figure 7.

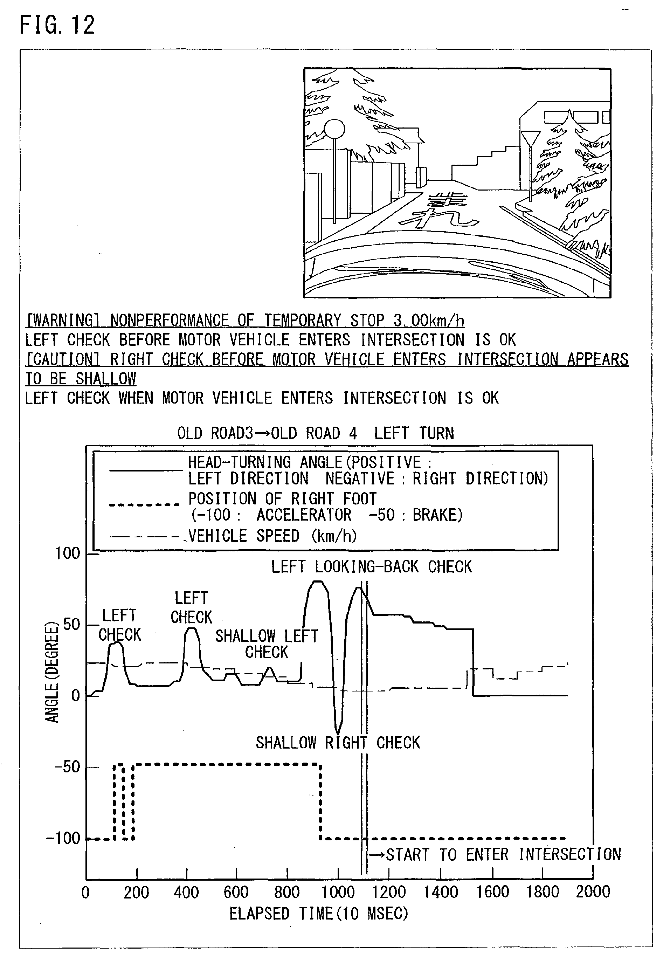

On the other hand, if "NO" in the step S71, an output of the evaluation result of the driving action with respect to the dangerous place is performed. In this embodiment, the evaluation result screen shown in Figure 12 is displayed on the display or printed on the paper by a printer.

More specifically, in a step S73, waveforms of the head-turning angle, the vehicle speed, and the position of the right foot (time variation) are depicted. Thus, a graph containing the respective waveforms shown at the lower part of the screen in Figure 12 is depicted.

Here, the longitudinal axis of the graph in Figure 12 represents an angle (degree), "0" is set at approximately the center, the upward direction is positive, and the downward direction is negative. If the head-turning angle is positive, a head turning to the left direction is indicated, and if the head-turning angle is negative, a head turning to the right direction is indicated. Furthermore, the lateral axis represents an elapsed time (10 msec), and the left end is set to 0 (time ta when the motor vehicle passes by 50 m before the dangerous place). Within the frames, the waveforms of the head-turning angle, the vehicle speed and the position of the right foot are depicted. Here, at the upper part of the frame, the name of the dangerous place is displayed. In Figure 12, the waveform of the head-turning angle is depicted by a solid line, the waveform of the vehicle speed is depicted by a dashed line, and the waveform of the position of the right foot is depicted by a dotted line. As to the position of the right foot, an adjustment is performed such that -100 of the scale of the angle indicates the side of the accelerator pedal 28, and -50 thereof indicates the side of the brake pedal 30. Furthermore, as to the vehicle speed, the scale of the angle directly indicates speeds (km/h). Here, although not represented in the drawing, the respective waveforms may be identified by being depicted by colors different from one another.

In a succeeding step S75, an estimated result of the behavior by the driver based on the waveform analysis is depicted in the graph as text information. Thus, what kind of checking motion is performed at what point is displayed. More specifically, from the analysis results in the steps S19 and S21, the executed right-and-left checking motion is specified, and a text indicating the direction and the depth of each checking motion is depicted at a position corresponding to the waveform of the head-turning angle. The text indicating the checking motion is displayed together with the waveform of the head-turning angle, that is, a comment indicating a right-and-left checking motion can be given to the graph of the waveform indicating the behavior of the head, and therefore, it is possible to easily grasp the right-and-left checking motion by the driver.

Furthermore, in a step S77, an estimated result of the state of the motor vehicle is depicted in the graph as text information. Thus, what point the motor vehicle enters in the dangerous place (intersection, etc.), etc. is displayed. In Figure 12, a double line vertically extending are depicted at a position corresponding to the time "tb" just at the dangerous place (immediate after entering), or the time "tb"' when the motor vehicle starts to enter the intersection. In addition, a text indicating "starting to enter the dangerous place (intersection)" is displayed. Together with the respective waveforms of the head-turning angle, the position of the right foot and the vehicle speed, the time when the motor vehicle enters the dangerous place is clearly indicated, and therefore, it is possible to easily grasp a difference and a change of the behavior before, immediately before, and the immediately after the dangerous place.

In a step S79, the text information indicating the estimated result of the right-and-left checking motion, the vehicle speed, and the position of the right foot is depicted. Thus, whether or not the accident preventive motion is properly performed is clearly shown, and if there is a point to be improved in the behavior of the driver from the viewpoints of a preventive safety, this matter is shown. In Figure 12, the texts indicating the evaluation result is displayed at the upper part of the graph. If a plurality of accident preventive motions are set to the dangerous place, the evaluated result is shown for each accident preventive motion in the set order. In example of Figure 12, the uppermost sentence is text information of "warning", and the "warning" is a determination result when an accident preventive motion is not performed. The text information of "warning" is displayed by red characters, for example. The accident preventive motion is a temporary stop, and the estimated result shows a failure of the temporary stop and the vehicle speed at that time. The second sentence from the uppermost is the text information of "OK". This shows that the left check before entering the intersection is properly performed. The text information of "OK" is displayed by blue characters, for example. The third sentence is the text information of "caution". This shows that the right check before entering the intersection is shallow. The text information of "caution" is displayed by orange-color characters, for example. The fourth sentence is the text information of "OK". This shows that the left check at a time of entering the intersection is properly performed.

In a step S81, a photograph of the dangerous place is depicted. The photography data is previously stored in the ROM or the HDD. In Figure 12, the photograph is shown at the upper right of the screen. Displaying the photography at the dangerous place can show how the dangerous place is dangerous, and thus can easily explain what kind of accident preventive motion is required.

In a step S83, the depicted evaluation result screen is output. In this embodiment, this is displayed on the display. Accordingly, the evaluation result screen as shown in Figure 12 is displayed. Thus, the user of the system 10 can easily grasp the driving action by the driver. The driver can confirm with at sight the time variation of his or her own driving action (right-and-left checking motion, position of the right foot, vehicle speed) with respect to the dangerous place by a waveform graph, and can easily know the correct or wrong of the accident preventive motion, the degree of attainment, the point to be improved, etc. by the text of the evaluation result. The evaluation result screen can give the driver a notice and thus is useful for education. Furthermore, an instructor of a driving school as well as drivers can easily grasp the driving action by the drivers and can precisely give an instruction, an advice, etc.

Here, in this embodiment, together with an evaluation result of the driving action by the driver with respect to the defined accident preventive motion, the waveform of the head-turning angle (behavior of the head), the waveform of the vehicle speed, and the waveform of the position of the right foot (behavior of the right toe) are displayed in the graph, but in another embodiment, only the evaluation result may be output. Additionally, on the graph, all the waveforms of the head-turning angle, the waveform of the vehicle speed, and the waveform of the position of the right foot are not necessarily output at the same time, and any one or any combination thereof may be output.

After completion of the step S83, the process proceeds to a step S85 to check the score of the driving action by the driver on the basis of the evaluation result.

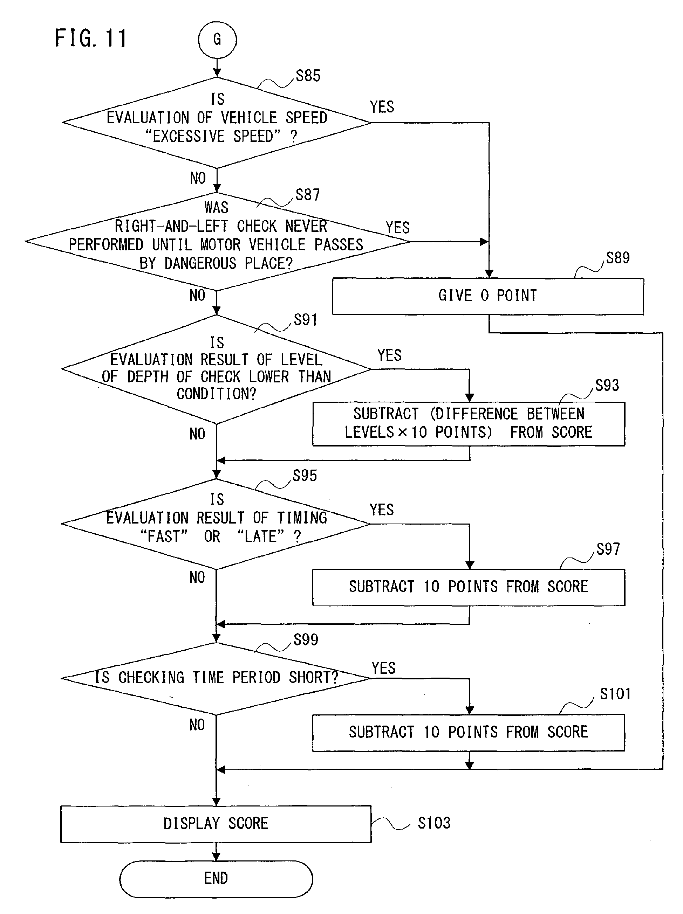

In the step S85 in Figure 11, it is determined whether or not the evaluation of the vehicle speed is "excessive speed". If "YES" in the step S85, the process proceeds to a step S89. On the other hand, if "NO" in the step S85, it is determined whether or not a right-and-left check was never performed until the motor vehicle passed by the dangerous place in a step S87. If "YES" in the step S87, that is, if the estimated result of the right-and-left checking motion is "no check", the process proceeds to the step S89.

In the step S89, 0 point is given to the score of the driver. In this system 10, whether or not a right-and-left checking motion is performed depending on a head-turning angle by the driver, and whether or not the gaze of the driver is directed to the right direction or the left direction is not strictly detected. When the vehicle speed is so fast at a time of performing the right-and-left check, the driver cannot view well, and therefore, the vehicle speed is required to be fully decelerated for the purpose of careful checking. Thus, in this embodiment, even if it is detected that a right and left head-turning motion is performed, if the vehicle speed is excessively high, it is not regarded that a fully visual check is performed. Accordingly, in a case of the excessive speed, irrespective of the results of other evaluation items, 0 point is given. By thus applying the vehicle speed to the evaluation item, whether or not a right and left head-turning motion by the driver is performed based on a natural right and left head-turning motion can be grasped. Thus, in a case that the system 10 is utilized for a driving lesson and reeducation, etc., a passing mark cannot be given to an artificial right and left check (pretention to check) by the driver (student). Furthermore, no check at a time of passing by the dangerous place is determined as a lack of an accident preventive intention by the driver, and in this case as well, 0 point is given irrespective of the results of the other evaluation items. After completion of the step S89, the process proceeds to a step S 103 .

On the other hand, if "NO" in the step S87, a demerit point system is adopted. Here, the initial value of the score is full marks (100 points, for example). More specifically, in a step S91, it is determined whether or not the evaluation result of the level of the depth of the check is lower than the condition. The level of the check is set in correspondence with the depth of the check, and in a case of a looking-back checking motion, the check level is 3, in a case of the normal checking motion, the check level is 2, in a case of a shallow checking motion, the check level is 1, and in a case of no check, the check level is 0. If "YES" in the step S91, that is, if the level of the depth of the check of the evaluation result is lower than the level of the depth of the check defined in the evaluation item, (the difference between the levels X a predetermined value (10 points, for example)) is subtracted from the score in a step S93.

In a case that "NO" in the step S91, the process proceeds to a step S95 as it is. In the step S95, it is determined whether the evaluation result of the timing is "fast" or "late". If "YES" in the step S95, a predetermined value (10 points, for example) is subtracted from the score in a step S97.

In a case that "NO" in the step S95, the process proceeds to a step S99 as it is. In the step S99, it is determined whether or not a checking time period is short. More specifically, it is determined whether or not the checking time period is less than a predetermined threshold value (0.3 seconds, for example). If "YES" in the step S99, a predetermined value (10 points, for example) is subtracted from the score in a step S101. In a case that "NO" in the step S99, the process proceeds to the step S103 as it is.

In the step S103, the score is output. In this embodiment, the score is displayed on the display. Thus, the score of the driving action by the driver with respect to the dangerous place is displayed. Here, in a case that a plurality of accident preventive motions are set to the dangerous place, the demerit point processing in the steps S91-S101 is performed for each accident preventive motion. Additionally, the score may be added up with scores with respect to other dangerous places on the course, and in this case, the score with respect to the course is acquired. The score is useful in managing a passing status, an educational process, a growth process, etc. of the student (driver) by a manager of education or reeducation.

The inventors et al. perform an on-road in-vehicle experiment in order to quantitatively evaluate the accuracy of the system 10. The experiment is performed to be aimed at participants of a taxi driver reeducation course (reeducation program of driving aimed at taxi drivers who caused an accident) held by the Yamasiro Driving School being an applicant of this invention. In the experiment, an instruction car of the driving school is used for the purpose of the safety, and the instructor rides on the passenger's side of the instruction car. The test subject is 23 professional taxi drivers (21 males, and 2 females). A single test subject or paired test subjects drive any one of the predetermined two courses (the course 1 is a public road of 10.9 km in Southern Kyoto Prefecture, and the course 2 is a public road of 3.6 km in Southern Kyoto Prefecture) decided according to a reeducation curse curriculum, being attached with the acceleration sensors 14 and 16.

Out of the data thus measured, focusing and analysis are especially performed on a section (A) in which a right turn is made at a signalized intersection to enter from a main road to a residential road, a section (B) in which a left turn is made at an unsignalized intersection to enter from a main road to a residential road, a section (C) in which a left turn is made at an unsignalized intersection from one residential road to another residential road, a section (D) in which a right turn is made at an unsignalized intersection from one residential road to another residential road, and a section (E) in which driving straight is made at a busy unsignalized intersection. Here, these 5 sections are also places where the instructor carefully observes motions of the driver in the reeducation curse.

First, the instructor is made to visually check the full length of the video image recording the situation of the experiments and to evaluate the driving actions of the 23 test subjects at each point on the basis of the evaluation items. Here, in the current experiment, a total number of the driving actions as an object to be analyzed is 85. Out of the 85 actions, the 25 actions are determined to be a good driving by the instructor, and the 60 actions are determined to be driving actions that requires improvements, that is, lacks any one of the minimum accident preventive motions to be performed, or do not completely satisfy the reference of the above-described evaluation items of (1)-(7) even though they are performed.

Next, how the result of the automatic evaluation by the system 10 matches the evaluation by the instructor is studied. In this system 10, the 24 actions out of all the 85 actions are determined to be good actions, and the 22 actions out of it matches the evaluation by the instructor (matching ratio 91.7%, reproducing ratio 88.0%). Similarly, the 61 actions are determined to be driving actions that require improvements by the system 10, and out of it, there are the 51 actions for which information as to which evaluation item out of the evaluation items (1)-(7) is not satisfied (what is a point to be improved) completely matches with the instructor (matching ratio 83.6%, reproducing ratio 85.0%).

Thus, it is confirmed that from the experiments, an evaluation matching the evaluation result by the instructor with accuracy equal to or more than 80% can be performed according to the system 10.

In the above-described embodiment, as an evaluation item for the accident preventive motion, the position of the right foot is included, and as to the accident preventive motion that does not require the evaluation of the position of the right foot, the evaluation item of the position of the right foot is handled as no definition, but in another embodiment, as an evaluation item, the accident preventive motion that does not include the position of the right foot may be defined. The dangerous place where the evaluation of the position of the right foot is required is a place where a motor vehicle can typically go straight and enough deceleration is not required during driving. In such a dangerous place, a braking stance has to be taken, and the evaluation item of the position of the right foot is required. However, in a case that the places that all require a right turn or a left turn are required are adopted as dangerous places on the course, the evaluation item of the position of the right foot need not be provided.

Furthermore, in each the above-described embodiments, the angular velocity sensors 14 and 16 are used in order to detect the movement of the head and the right toe of the driver, but in another embodiment, another detection device may be utilized. For example, it may be possible to use an acceleration sensor. In correspondence with the right-and-left checking motion and a movement of the right foot of the driver, the acceleration changes, and therefore, by analyzing the acceleration data of the head and the acceleration of the right toe as movement data, the right-and-left checking motion and the position of the right foot can be analyzed.

In each of the above-described embodiment, the driving action by the driver with respect to the dangerous place is evaluated, but the place to which the system 10 is applied is not restricted to the dangerous place, such as an accident-prone place. That is, in this system 10, it becomes possible to evaluate the driving action by the driver with respect to a predetermined place, and in another embodiment, a general intersection and a proper place, for example, may be adopted as a predetermined place.

In addition, in the above-described embodiment, the name, the coordinates, and the feature, etc. of the dangerous place and the dangerous preventive motion defining data are previously stored in the memory (not illustrated) of the computer for analysis 22 as dangerous place information shown in Figure 5, but the dangerous place information can be adequately updated thereafter. For example, it may be conceivable that by an inter-vehicle communication, such as a VICS (Vehicle Information and Communication System), the name, the coordinates, the feature, etc. of the dangerous place and the dangerous preventive motion defining data are received, and by the received data, the aforementioned memory is rewritten (updated). For example, in a case that a place under construction is newly set, the latitude and the longitude of the place may be additively registered as dangerous place data, and a danger preventive motion (driving action to avoid the place by driving the opposite lane, for example) of the dangerous place may be additively registered as definition data. Here, another broadcast system may be adopted without being restricted to the VICS. For example, using a taxi wireless system and a wireless LAN are conceivable.

Although the present invention has been described and illustrated in detail, it is clearly understood that the same is by way of illustration and example only and is not to be taken by way of limitation, the scope of the present invention being limited only by the terms of the appended claims.

## Claims

1. A driving action automatic evaluating system to evaluate a driving action of a driver of a motor vehicle with respect to a predetermined place, comprising:
a head angular velocity sensor (14) attached to a head of said driver arranged to measure angular velocity data of the head of said driver;
a foot angular velocity sensor (16) attached to the right foot of said driver arranged to measure angular velocity data of the right foot of said driver;
a position detector (20) arranged to detect position data of said motor vehicle;
a recorder (12) arranged to record at least said angular velocity data detected by said head angular velocity sensor, said angular velocity data detected by said foot angular velocity sensor, and said position data of said motor vehicle detected by said position detector;
a motion defining data storage arranged to store motion defining data defining a minimum accident preventive motion to be performed for each predetermined place, wherein the motion defining data for a said predetermined place comprises data relating to at least a right-and-left checking motion of the head of the driver, a right foot position of the driver, and a vehicle speed for that predetermined place, wherein the right-and-left checking motion for a said predetermined place comprises a respective predetermined visual checking motion for that predetermined place, wherein the right foot position of the driver indicates which pedal the right foot of the driver should be on for that predetermined place;
a controller arranged to use said angular velocity data detected by said head angular velocity sensor (14) to determine a right-and-left motion of the head of the driver, to use said angular velocity data detected by said foot angular velocity sensor (16) to determine a right foot position of the driver, and to determine the vehicle speed of the motor vehicle;
an evaluator arranged to evaluate whether or not said accident preventive motion is properly performed by the driver to determine an evaluation result for each predetermined place, wherein a said evaluation result for a said predetermined place is determined by comparing said determined right-and-left motion of the head of the driver at said predetermined place with the data in the motion defining data relating to the right-and-left checking motion of the head of the driver for said predetermined place, by comparing said determined right foot position of the driver at said predetermined place with the data in the motion defining data relating to the right foot position of the driver for said predetermined place, and by comparing for said determined vehicle speed at said predetermined place with the data in the motion defining data relating to vehicle speed for said predetermined place; and
an outputter arranged to output an evaluation result by said evaluator.

2. A driving action automatic evaluating system according to claim 1, wherein said outputter is further arranged to output a waveform indicating a behavior of said head based on at least said angular velocity data of the head of said driver.

3. A driving action automatic evaluating system according to claim 1 or 2, wherein said outputter is further arranged to output a waveform indicating a behavior of the position of the right foot based on said angular velocity data detected by said foot angular velocity sensor (16).

4. A driving action automatic evaluating system according to any one of claims 1 to 3, wherein said motion defining data storage is arranged to store data indicating a check direction, the number of checks, a depth of a check, a checking time period, and a checking timing as the evaluation items in relation to said right-and-left checking motion.

5. A driving action automatic evaluating system according to claim 2, wherein said outputter is arranged to indicate an estimated right-and-left checking motion as text information above the waveform indicating the behavior of said head.

6. A driving action automatic evaluating system according to any one of claims 1 to 5, wherein said outputter is further arranged to output a waveform indicating a vehicle speed based on said position data.

7. A driving action automatic evaluating system according to claim 2, 3 or 6, wherein said outputter is arranged to output text information indicating a situation of the motor vehicle estimated based on said position data above said waveform.

8. A driving action automatic evaluating system according to any one of claims 1 to 7, further comprising a scorer arranged to give a score on the basis of an evaluation by said evaluator, wherein
said scorer is arranged to give o point to the driver when the vehicle speed does not satisfy said motion defining data, or when no right-and-left checking motion is performed before passing by said predetermined place.

9. An evaluation program stored in a storage medium readable by a computer of a driving action automatic evaluating system for evaluating a driving action by a driver of a motor vehicle with respect to a predetermined place causes said computer to execute:
a head angular velocity detecting step for detecting angular velocity data from a head angular velocity sensor (14) attached to a head of said driver;
a foot angular velocity detecting step for detecting angular velocity data from a foot angular velocity sensor (16) attached to a foot of said driver;
a position detecting step for detecting position data of said motor vehicle;
a recording step for recording at least said angular velocity data detected in said head angular velocity detecting step, said angular velocity data detected in said foot angular velocity detecting step, and said position data of said motor vehicle detected in said position detecting step;
a motion defining data storing step for storing motion defining data defining a minimum accident preventive motion to be performed for each predetermined place, wherein the motion defining data for a said predetermined place comprises data relates to at least a right-and-left checking motion of the head of the driver, a right foot position of the driver, and a vehicle speed for that predetermined place, wherein the right-and-left checking motion for a said predetermined place comprises a respective predetermined visual checking motion for that predetermined place, wherein the right foot position of the driver indicates which pedal the right foot of the driver should be on for that predetermined place;
a determining step for determining a right-and-left motion of the head of the driver, for determining a right foot position of the driver, and for determining the vehicle speed of the motor vehicle;
an evaluating step for evaluating whether or not said accident preventive motion is properly performed by the driver to determine an evaluation result for each predetermined place, wherein a said evaluation result for a said predetermined place is determined by comparing said determined right-and-left motion of the head of the driver at said predetermined place with the data in the motion defining data relating to the right-and-left checking motion of the head of the driver for said predetermined place, by comparing said determined right foot position of the driver at said predetermined place with the data in the motion defining data relating to the right foot position of the driver for said predetermined place, and by comparing for said determined vehicle speed at said predetermined place with the data in the motion defining data relating to vehicle speed for said predetermined place; and
an outputting step for outputting an evaluation result from said evaluating step.

10. An driving action evaluating method for evaluating a driving action of a driver of a motor vehicle with respect to a predetermined place causes a computer to execute:
a head angular velocity detecting step for detecting angular velocity data from a head angular velocity sensor (14) attached to a head of said driver;
a foot angular velocity detecting step for detecting angular velocity data from a foot angular velocity sensor (16) attached to a foot of said driver;
a position detecting step for detecting position data of said motor vehicle using a position detector (20);
a recording step for recording at least said angular velocity data detected by said head angular velocity detector (14), said angular velocity data detected by said foot angular velocity sensor (16), and said position data of said motor vehicle detected by said position detector (20);
a motion defining data storing step for storing motion defining data defining a minimum accident preventive motion to be performed for each predetermined place, wherein the motion defining data for a said predetermined place comprises data relates to at least a right-and-left checking motion of the head of the driver, a right foot position of the driver, and a vehicle speed for that predetermined place, wherein the right-and-left checking motion for a said predetermined place comprises a respective predetermined visual checking motion for that predetermined place, wherein the right foot position of the driver indicates which pedal the right foot of the driver should be on for that predetermined place;
a determining step for determining a right-and-left motion of the head of the driver, for determining a right foot position of the driver, and for determining the vehicle speed of the motor vehicle;
an evaluating step for evaluating whether or not said accident preventive motion is properly performed by the driver to determine an evaluation result for each predetermined place, wherein a said evaluation result for a said predetermined place is determined by comparing said determined right-and-left motion of the head of the driver at said predetermined place with the data in the motion defining data relating to the right-and-left checking motion of the head of the driver for said predetermined place, by comparing said determined right foot position of the driver at said predetermined place with the data in the motion defining data relating to the right foot position of the driver for said predetermined place, and by comparing for said determined vehicle speed at said predetermined place with the data in the motion defining data relating to vehicle speed for said predetermined place; and
an outputting step for outputting an evaluation result by said evaluator.

## Patentansprüche

1. Automatisches Fahrhandlungs-Auswertungssystem zur Auswertung einer Fahrhandlung eines Fahrers eines Kraftfahrzeugs in Bezug auf einen vorbestimmten Ort, umfassend:
einen am Kopf des genannten Fahrers angebrachten Kopf-Winkelgeschwindigkeitssensor (14), der zum Messen von Winkelgeschwindigkeitsdaten des Kopfes des genannten Fahrers angeordnet ist,
einen am rechten Fuß des genannten Fahrers angebrachten Fuß-Winkelgeschwindigkeitssensor (16), der zum Messen von Winkelgeschwindigkeitsdaten des rechten Fußes des genannten Fahrers angeordnet ist,
einen Positionsdetektor (20), der zum Erfassen von Positionsdaten des genannten Kraftfahrzeugs angeordnet ist,
eine Aufzeichnungseinrichtung (12), die zum Aufzeichnen wenigstens der von dem genannten Kopf-Winkelgeschwindigkeitssensor erfassten genannten Winkelgeschwindigkeitsdaten, der von dem genannten Fuß-Winkelgeschwindigkeitssensor erfassten genannten Winkelgeschwindigkeitsdaten und der von dem genannten Positionsdetektor erfassten genannten Positionsdaten des genannten Kraftfahrzeugs angeordnet ist,
einen Speicher für bewegungsdefinierende Daten, der zum Speichern von bewegungsdefinierenden Daten angeordnet ist, die eine Mindest-Unfallverhütungsbewegung definieren, die für jeden vorbestimmten Ort durchzuführen ist, wobei die bewegungsdefinierenden Daten für einen genannten vorbestimmten Ort Daten umfassen, die sich wenigstens auf eine Rechts-Links-Prüfbewegung des Kopfes des Fahrers, eine Position des rechten Fußes des Fahrers und eine Fahrzeuggeschwindigkeit für diesen vorbestimmten Ort beziehen, wobei die Rechts-Links-Prüfbewegung für einen genannten vorbestimmten Ort eine jeweilige vorbestimmte Sichtprüfbewegung für diesen vorbestimmten Ort umfasst, wobei die Position des rechten Fußes des Fahrers anzeigt, auf welchem Pedal der rechte Fuß des Fahrers für diesen vorbestimmten Ort sein sollte,
einen Controller, der angeordnet ist zur Verwendung der genannten von dem genannten Kopf-Winkelgeschwindigkeitssensor (14) erfassten Winkelgeschwindigkeitsdaten zum Ermitteln einer Rechts-Links-Bewegung des Kopfes des Fahrers, zur Verwendung der von dem genannten Fuß-Winkelgeschwindigkeitssensor (16) erfassten genannten Winkelgeschwindigkeitsdaten zum Ermitteln einer Position des rechten Fußes des Fahrers und zum Ermitteln der Fahrzeuggeschwindigkeit des Kraftfahrzeugs,
eine Auswertungseinheit, die angeordnet ist zur Auswertung, ob die genannte Unfallverhütungsbewegung vom Fahrer ordnungsgemäß durchgeführt wird oder nicht, um ein Auswertungsergebnis für jeden vorbestimmten Ort zu ermitteln, wobei ein genanntes Auswertungsergebnis für einen genannten vorbestimmten Ort ermittelt wird durch Vergleichen der genannten ermittelten Rechts-Links-Bewegung des Kopfes des Fahrers an dem genannten vorbestimmten Ort mit den Daten in den bewegungsdefinierenden Daten, die sich auf die Rechts-Links-Prüfbewegung des Kopfes des Fahrers für den genannten vorbestimmten Ort beziehen, durch Vergleichen der genannten vorbestimmten Position des rechten Fußes des Fahrers an dem genannten vorbestimmten Ort mit den Daten in den bewegungsdefinierenden Daten, die sich auf die Position des rechten Fußes des Fahrers für den genannten vorbestimmten Ort beziehen, und durch Vergleichen der genannten ermittelten Fahrzeuggeschwindigkeit an dem genannten vorbestimmten Ort mit den Daten in den bewegungsdefinierenden Daten, die sich auf die Fahrzeuggeschwindigkeit für den genannten vorbestimmten Ort beziehen, und
eine Ausgabeeinheit, die angeordnet ist zum Ausgeben eines Auswertungsergebnisses durch die genannte Auswertungseinheit.

2. Automatisches Fahrhandlungs-Auswertungssystem nach Anspruch 1, wobei die genannte Ausgabeeinheit ferner angeordnet ist, um eine Wellenform auszugeben, die ein Verhalten des genannten Kopfes auf Basis von wenigstens der genannten Winkelgeschwindigkeitsdaten des Kopfes des genannten Fahrers anzeigt.

3. Automatisches Fahrhandlungs-Auswertungssystem nach Anspruch 1 oder 2, wobei die genannte Ausgabeeinheit ferner angeordnet ist, um eine Wellenform auszugeben, die ein Verhalten der Position des rechten Fußes auf Basis der genannten von dem genannten Fuß-Winkelgeschwindigkeitssensor (16) erfassten Winkelgeschwindigkeitsdaten anzeigt.

4. Automatisches Fahrhandlungs-Auswertungssystem nach einem der Ansprüche 1 bis 3, wobei der genannte Speicher für bewegungsdefinierende Daten angeordnet ist, um Daten zu speichern, die eine Prüfrichtung, die Anzahl von Prüfungen, eine Prüfungstiefe, eine Prüfungszeitspanne und einen Prüfungszeitpunkt als die Auswertungselemente in Bezug auf die genannte Rechts-Links-Prüfbewegung anzeigen.

5. Automatisches Fahrhandlungs-Auswertungssystem nach Anspruch 2, wobei die genannte Ausgabeeinheit angeordnet ist zum Anzeigen einer geschätzten Rechts-Links-Prüfbewegung als Textinformationen über der Wellenform, die das Verhalten des genannten Kopfes anzeigt.

6. Automatisches Fahrhandlungs-Auswertungssystem nach einem der Ansprüche 1 bis 5, wobei die genannte Ausgabeeinheit ferner angeordnet ist zum Ausgeben einer Wellenform, die eine Fahrzeuggeschwindigkeit auf Basis der genannten Positionsdaten anzeigt.

7. Automatisches Fahrhandlungs-Auswertungssystem nach Anspruch 2, 3 oder 6, wobei die genannte Ausgabeeinheit angeordnet ist zum Ausgeben von Textinformationen, die eine auf Basis der genannten Positionsdaten über der genannten Wellenform geschätzte Situation des Kraftfahrzeugs anzeigt.

8. Automatisches Fahrhandlungs-Auswertungssystem nach einem der Ansprüche 1 bis 7, das ferner eine Bewertungseinheit aufweist, der angeordnet ist zum Vergeben einer Bewertung auf der Basis einer Auswertung durch die genannte Auswertungseinheit, wobei
die genannte Bewertungseinheit angeordnet ist, um dem Fahrer 0 Punkte zu geben, wenn die Fahrzeuggeschwindigkeit den genannten bewegungsdefinierenden Daten nicht genügen oder wenn keine Rechts-Links-Prüfbewegung durchgeführt wird, bevor an dem genannten vorbestimmten Ort vorbeigefahren wird.

9. Auswertungsprogramm, in einem Speichermedium gespeichert, welches durch einen Computer eines automatischen Fahrhandlungs-Auswertungssystems zur Auswertung einer Fahrhandlung durch einen Fahrer eines Kraftfahrzeugs in Bezug auf einen vorbestimmten Ort lesbar ist, das den genannten Computer zum Ausführen von Folgendem veranlasst:
einem Kopf-Winkelgeschwindigkeitserfassungsschritt zum Erfassen von Winkelgeschwindigkeitsdaten von einem am Kopf des genannten Fahrers angebrachten Kopf-Winkelgeschwindigkeitssensor (14),
einem Fuß-Winkelgeschwindigkeitserfassungsschritt zum Erfassen von Winkelgeschwindigkeitsdaten von einem an einem Fuß des genannten Fahrers angebrachten Fuß-Winkelgeschwindigkeitssensor (16),
einem Positionserfassungsschritt zum Erfassen von Positionsdaten des genannten Kraftfahrzeugs,
einem Aufzeichnungsschritt zum Aufzeichnen wenigstens von den in dem genannten Kopf-Winkelgeschwindigkeitserfassungsschritt erfassten genannten Winkelgeschwindigkeitsdaten, den in dem genannten Fuß-Winkelgeschwindigkeitserfassungsschritt erfassten genannten Winkelgeschwindigkeitsdaten und den in dem genannten Positionserfassungsschritt erfassten Positionsdaten des genannten Kraftfahrzeugs,
einem Schritt des Speicherns von bewegungsdefinierenden Daten zum Speichern von bewegungsdefinierenden Daten, die eine Mindest-Unfallverhütungsbewegung definieren, die für jeden vorbestimmten Ort durchzuführen ist, wobei die bewegungsdefinierenden Daten für einen genannten vorbestimmten Ort Daten umfassen, die sich wenigstens auf eine Rechts-Links-Prüfbewegung des Kopfes des Fahrers, eine Position des rechten Fußes des Fahrers und eine Fahrzeuggeschwindigkeit für diesen vorbestimmten Ort beziehen, wobei die Rechts-Links-Prüfbewegung für einen genannten vorbestimmten Ort eine jeweilige vorbestimmte Sichtprüfungsbewegung für diesen vorbestimmten Ort umfasst, wobei die Position des rechten Fußes des Fahrers anzeigt, auf welchem Pedal der rechte Fuß des Fahrers für diesen vorbestimmten Ort sein sollte,
einem Ermittlungsschritt zum Ermitteln einer Rechts-Links-Bewegung des Kopfes des Fahrers, zum Ermitteln einer Position des rechten Fußes des Fahrers und zum Ermitteln der Fahrzeuggeschwindigkeit des Kraftfahrzeugs,
einem Auswertungsschritt zur Auswertung, ob die genannte Unfallverhütungsbewegung vom Fahrer ordnungsgemäß durchgeführt wird oder nicht, um ein Auswertungsergebnis für jeden vorbestimmten Ort zu ermitteln, wobei ein genanntes Auswertungsergebnis für einen genannten vorbestimmten Ort ermittelt wird durch Vergleichen der genannten ermittelten Rechts-Links-Bewegung des Kopfes des Fahrers an dem genannten vorbestimmten Ort mit den Daten in den bewegungsdefinierenden Daten, die sich auf die Rechts-Links-Prüfbewegung des Kopfes des Fahrers für den genannten vorbestimmten Ort beziehen, durch Vergleichen der genannten vorbestimmten Position des rechten Fußes des Fahrers an dem genannten vorbestimmten Ort mit den Daten in den bewegungsdefinierenden Daten, die sich auf die Position des rechten Fußes des Fahrers für den genannten vorbestimmten Ort beziehen, und durch Vergleichen der genannten ermittelten Fahrzeuggeschwindigkeit an dem genannten vorbestimmten Ort mit den Daten in den bewegungsdefinierenden Daten, die sich auf die Fahrzeuggeschwindigkeit für den genannten vorbestimmten Ort beziehen, und
einem Ausgabeschritt zum Ausgeben eines Auswertungsergebnisses aus dem genannten Auswertungsschritt.

10. Fahrhandlungs-Auswertungsverfahren zur Auswertung einer Fahrhandlung eines Fahrers eines Kraftfahrzeugs in Bezug auf einen vorbestimmten Ort, das einen Computer zum Ausführen von Folgendem veranlasst:
einem Kopf-Winkelgeschwindigkeitserfassungsschritt zum Erfassen von Winkelgeschwindigkeitsdaten von einem am Kopf des genannten Fahrers angebrachten Kopf-Winkelgeschwindigkeitssensor (14),
einem Fuß-Winkelgeschwindigkeitserfassungsschritt zum Erfassen von Winkelgeschwindigkeitsdaten von einem an einem Fuß des genannten Fahrers angebrachten Fuß-Winkelgeschwindigkeitssensor (16),
einem Positionserfassungsschritt zum Erfassen von Positionsdaten des genannten Kraftfahrzeugs mithilfe eines Positionsdetektors (20),
einem Aufzeichnungsschritt zum Aufzeichnen wenigstens der von dem genannten Kopf-Winkelgeschwindigkeitssensor (14) erfassten genannten Winkelgeschwindigkeitsdaten, der von dem genannten Fuß-Winkelgeschwindigkeitssensor (16) erfassten genannten Winkelgeschwindigkeitsdaten und der von dem genannten Positionsdetektor (20) erfassten genannten Positionsdaten des genannten Kraftfahrzeugs,
einem Schritt des Speicherns von bewegungsdefinierenden Daten zum Speichern von bewegungsdefinierenden Daten, die eine Mindest-Unfallverhütungsbewegung definieren, die für jeden vorbestimmten Ort durchzuführen ist, wobei die bewegungsdefinierenden Daten für einen genannten vorbestimmten Ort Daten umfassen, die sich wenigstens auf eine Rechts-Links-Prüfbewegung des Kopfes des Fahrers, eine Position des rechten Fußes des Fahrers und eine Fahrzeuggeschwindigkeit für diesen vorbestimmten Ort beziehen, wobei die Rechts-Links-Prüfbewegung für einen genannten vorbestimmten Ort eine jeweilige vorbestimmte Sichtprüfungsbewegung für diesen vorbestimmten Ort umfasst, wobei die Position des rechten Fußes des Fahrers anzeigt, auf welchem Pedal der rechte Fuß des Fahrers für diesen vorbestimmten Ort sein sollte,
einem Ermittlungsschritt zum Ermitteln einer Rechts-Links-Bewegung des Kopfes des Fahrers, zum Ermitteln einer Position des rechten Fußes des Fahrers und zum Ermitteln der Fahrzeuggeschwindigkeit des Kraftfahrzeugs,
einem Auswertungsschritt zur Auswertung, ob die genannte Unfallverhütungsbewegung vom Fahrer ordnungsgemäß durchgeführt wird oder nicht, um ein Auswertungsergebnis für jeden vorbestimmten Ort zu ermitteln, wobei ein genanntes Auswertungsergebnis für einen genannten vorbestimmten Ort ermittelt wird durch Vergleichen der genannten ermittelten Rechts-Links-Bewegung des Kopfes des Fahrers an dem genannten vorbestimmten Ort mit den Daten in den bewegungsdefinierenden Daten, die sich auf die Rechts-Links-Prüfbewegung des Kopfes des Fahrers für den genannten vorbestimmten Ort beziehen, durch Vergleichen der genannten vorbestimmten Position des rechten Fußes des Fahrers an dem genannten vorbestimmten Ort mit den Daten in den bewegungsdefinierenden Daten, die sich auf die Position des rechten Fußes des Fahrers für den genannten vorbestimmten Ort beziehen, und durch Vergleichen der genannten ermittelten Fahrzeuggeschwindigkeit an dem genannten vorbestimmten Ort mit den Daten in den bewegungsdefinierenden Daten, die sich auf die Fahrzeuggeschwindigkeit für den genannten vorbestimmten Ort beziehen, und
einem Ausgabeschritt zum Ausgeben eines Auswertungsergebnisses durch die genannte Auswertungseinheit.

## Revendications

1. Système d'évaluation automatique d'action de conduite servant à évaluer une action de conduite d'un conducteur d'un véhicule motorisé par rapport à un endroit prédéterminé, comportant :
un capteur de vitesse angulaire de tête (14) attaché au niveau de la tête dudit conducteur et agencé pour mesurer des données de vitesse angulaire de la tête dudit conducteur ;
un capteur de vitesse angulaire de pied (16) attaché au niveau du pied droit dudit conducteur et agencé pour mesurer des données de vitesse angulaire du pied droit dudit conducteur ;
un détecteur de position (20) agencé pour détecter des données de position dudit véhicule motorisé ;
un enregistreur (12) agencé pour enregistrer au moins lesdites données de vitesse angulaire détectées par ledit capteur de vitesse angulaire de tête, lesdites données de vitesse angulaire détectées par ledit capteur de vitesse angulaire de pied, et lesdites données de position dudit véhicule motorisé détectées par ledit détecteur de position ;
un dispositif de stockage de données de définition de mouvement agencé pour stocker des données de définition de mouvement définissant un mouvement minimum de prévention d'accident devant être effectué pour chaque endroit prédéterminé, dans lequel les données de définition de mouvement pour un dit endroit prédéterminé comportent des données se rapportant à au moins un mouvement de contrôle vers la droite et la gauche de la tête du conducteur, une position du pied droit du conducteur, et une vitesse du véhicule pour cet endroit prédéterminé, dans lequel le mouvement de contrôle vers la droite et la gauche pour un dit endroit prédéterminé comporte un mouvement de contrôle visuel prédéterminé respectif pour cet endroit prédéterminé, dans lequel la position du pied droit du conducteur indique sur quelle pédale doit se trouver le pied droit du conducteur pour cet endroit prédéterminé ;
un dispositif de commande agencé pour utiliser lesdites données de vitesse angulaire détectées par ledit capteur de vitesse angulaire de tête (14) pour déterminer un mouvement vers la droite et la gauche de la tête du conducteur, pour utiliser lesdites données de vitesse angulaire détectées par ledit capteur de vitesse angulaire de pied (16) pour déterminer une position du pied droit du conducteur, et pour déterminer la vitesse du véhicule motorisé ;
un dispositif évaluateur agencé pour évaluer si oui ou non ledit mouvement de prévention d'accident est effectué correctement par le conducteur pour déterminer un résultat d'évaluation pour chaque endroit prédéterminé, dans lequel un dit résultat d'évaluation pour un dit endroit prédéterminé est déterminé en comparant ledit mouvement déterminé vers la droite et la gauche de la tête du conducteur au niveau dudit endroit prédéterminé par rapport aux données dans les données de définition de mouvement se rapportant au mouvement de contrôle vers la droite et la gauche de la tête du conducteur pour ledit endroit prédéterminé, en comparant ladite position déterminée du pied droit du conducteur au niveau dudit endroit prédéterminé par rapport aux données dans les données de définition de mouvement se rapportant à la position du pied droit du conducteur pour ledit endroit prédéterminé, et en comparant ladite vitesse déterminée du véhicule au niveau dudit endroit prédéterminé par rapport aux données dans les données de définition de mouvement se rapportant à la vitesse du véhicule pour ledit endroit prédéterminé ; et
un dispositif émetteur agencé pour émettre un résultat d'évaluation par ledit dispositif évaluateur.

2. Système d'évaluation automatique d'action de conduite selon la revendication 1, dans lequel ledit dispositif émetteur est par ailleurs agencé pour émettre une forme d'onde indiquant un comportement de ladite tête en fonction au moins desdites données de vitesse angulaire de la tête dudit conducteur.

3. Système d'évaluation automatique d'action de conduite selon la revendication 1 ou la revendication 2, dans lequel ledit dispositif émetteur est par ailleurs agencé pour émettre une forme d'onde indiquant un comportement de la position du pied droit en fonction desdites données de vitesse angulaire détectées par ledit capteur de vitesse angulaire de pied (16).

4. Système d'évaluation automatique d'action de conduite selon l'une quelconque des revendications 1 à 3, dans lequel ledit dispositif de stockage de données de définition de mouvement est agencé pour stocker des données indiquant une direction de contrôle, le nombre de contrôles, une profondeur d'un contrôle, une durée de contrôle, et une temporisation de contrôle comme éléments d'évaluation se rapportant audit mouvement de contrôle vers la droite et la gauche.

5. Système d'évaluation automatique d'action de conduite selon la revendication 2, dans lequel ledit dispositif émetteur est agencé pour indiquer un mouvement estimé de contrôle vers la droite et la gauche sous forme d'informations de texte au-dessus de la forme d'onde pour indiquer le comportant de ladite tête.

6. Système d'évaluation automatique d'action de conduite selon l'une quelconque des revendications 1 à 5, dans lequel ledit dispositif émetteur est par ailleurs agencé pour émettre une forme d'onde indiquant une vitesse de véhicule en fonction desdites données de position.

7. Système d'évaluation automatique d'action de conduite selon la revendication 2, la revendication 3 ou la revendication 6, dans lequel ledit dispositif émetteur est agencé pour émettre des informations de texte indiquant une situation du véhicule motorisé estimée en fonction desdites données de position au-dessus de ladite forme d'onde.

8. Système d'évaluation automatique d'action de conduite selon l'une quelconque des revendications 1 à 7, comportant par ailleurs un dispositif marqueur agencé pour donner un score en fonction d'une évaluation par ledit dispositif évaluateur, dans lequel
ledit dispositif marqueur est agencé pour donner 0 point au conducteur quand la vitesse du véhicule ne satisfait pas auxdites données de définition de mouvement, ou quand aucun mouvement de contrôle vers la droite et la gauche n'est effectué avant de passer par ledit endroit prédéterminé.

9. Programme d'évaluation stocké dans un support de stockage lisible par un ordinateur d'un système d'évaluation automatique d'action de conduite servant à évaluer une action de conduite par un conducteur d'un véhicule motorisé par rapport à un endroit prédéterminé, amenant ledit ordinateur à exécuteur :
une étape de détection de vitesse angulaire de tête permettant de détecter des données de vitesse angulaire en provenance d'un capteur de vitesse angulaire de tête (14) attaché au niveau de la tête dudit conducteur ;
une étape de détection de vitesse angulaire de pied permettant de détecter des données de vitesse angulaire en provenance d'un capteur de vitesse angulaire de pied (16) attaché au niveau d'un pied dudit conducteur ;
une étape de détection de position permettant de détecter des données de position dudit véhicule motorisé ;
une étape d'enregistrement permettant d'enregistrer au moins lesdites données de vitesse angulaire détectées au cours de ladite étape de détection de vitesse angulaire de tête, lesdites données de vitesse angulaire détectées au cours de ladite étape de détection de vitesse angulaire de pied, et lesdites données de position dudit véhicule motorisé détectées au cours de ladite étape de détection de position ;
une étape de stockage de données de définition de mouvement permettant de stocker des données de définition de mouvement définissant un mouvement minimum de prévention d'accident devant être effectué pour chaque endroit prédéterminé, dans lequel les données de définition de mouvement pour un dit endroit prédéterminé comportent des données se rapportant à au moins un mouvement de contrôle vers la droite et la gauche de la tête du conducteur, une position du pied droit du conducteur, et une vitesse du véhicule pour cet endroit prédéterminé, dans lequel le mouvement de contrôle vers la droite et la gauche pour un dit endroit prédéterminé comporte un mouvement de contrôle visuel prédéterminé respectif pour cet endroit prédéterminé, dans lequel la position du pied droit du conducteur indique sur quelle pédale doit se trouver le pied droit du conducteur pour cet endroit prédéterminé ;
une étape de détermination permettant de déterminer un mouvement vers la droite et la gauche de la tête du conducteur, de déterminer une position du pied droit du conducteur, et de déterminer la vitesse du véhicule motorisé ;
une étape d'évaluation permettant d'évaluer si oui ou non ledit mouvement de prévention d'accident est effectué correctement par le conducteur pour déterminer un résultat d'évaluation pour chaque endroit prédéterminé, dans lequel un dit résultat d'évaluation pour un dit endroit prédéterminé est déterminé en comparant ledit mouvement déterminé vers la droite et la gauche de la tête du conducteur au niveau dudit endroit prédéterminé par rapport aux données dans les données de définition de mouvement se rapportant au mouvement de contrôle vers la droite et la gauche de la tête du conducteur pour ledit endroit prédéterminé, en comparant ladite position déterminée du pied droit du conducteur au niveau dudit endroit prédéterminé par rapport aux données dans les données de définition de mouvement se rapportant à la position du pied droit du conducteur pour ledit endroit prédéterminé, et en comparant ladite vitesse déterminée du véhicule au niveau dudit endroit prédéterminé par rapport aux données dans les données de définition de mouvement se rapportant à la vitesse du véhicule pour ledit endroit prédéterminé ; et
une étape d'émission permettant d'émettre un résultat d'évaluation provenant de ladite étape d'évaluation.

10. Procédé d'évaluation d'action de conduite servant à évaluer une action de conduite d'un conducteur d'un véhicule motorisé par rapport à un endroit prédéterminé amenant un ordinateur à exécuteur :
une étape de détection de vitesse angulaire de tête permettant de détecter des données de vitesse angulaire en provenance d'un capteur de vitesse angulaire de tête (14) attaché au niveau de la tête dudit conducteur ;
une étape de détection de vitesse angulaire de pied permettant de détecter des données de vitesse angulaire en provenance d'un capteur de vitesse angulaire de pied (16) attaché au niveau d'un pied dudit conducteur ;
une étape de détection de position permettant de détecter des données de position dudit véhicule motorisé au moyen d'un détecteur de position (20) ;
une étape d'enregistrement permettant d'enregistrer au moins lesdites données de vitesse angulaire détectées par ledit détecteur de vitesse angulaire de tête (14), lesdites données de vitesse angulaire détectées par ledit capteur de vitesse angulaire de pied (16), et lesdites données de position dudit véhicule motorisé détectées par ledit détecteur de position (20) ;
une étape de stockage de données de définition de mouvement permettant de stocker des données de définition de mouvement définissant un mouvement minimum de prévention d'accident devant être effectué pour chaque endroit prédéterminé, dans lequel les données de définition de mouvement pour un dit endroit prédéterminé comportent des données se rapportant à au moins un mouvement de contrôle vers la droite et la gauche de la tête du conducteur, une position du pied droit du conducteur, et une vitesse du véhicule pour cet endroit prédéterminé, dans lequel le mouvement de contrôle vers la droite et la gauche pour un dit endroit prédéterminé comporte un mouvement de contrôle visuel prédéterminé respectif pour cet endroit prédéterminé, dans lequel la position du pied droit du conducteur indique sur quelle pédale doit se trouver le pied droit du conducteur pour cet endroit prédéterminé ;
une étape de détermination permettant de déterminer un mouvement vers la droite et la gauche de la tête du conducteur, de déterminer une position du pied droit du conducteur, et de déterminer la vitesse du véhicule motorisé ;
une étape d'évaluation permettant d'évaluer si oui ou non ledit mouvement de prévention d'accident est effectué correctement par le conducteur pour déterminer un résultat d'évaluation pour chaque endroit prédéterminé, dans lequel un dit résultat d'évaluation pour un dit endroit prédéterminé est déterminé en comparant ledit mouvement déterminé vers la droite et la gauche de la tête du conducteur au niveau dudit endroit prédéterminé par rapport aux données dans les données de définition de mouvement se rapportant au mouvement de contrôle vers la droite et la gauche de la tête du conducteur pour ledit endroit prédéterminé, en comparant ladite position déterminée du pied droit du conducteur au niveau dudit endroit prédéterminé par rapport aux données dans les données de définition de mouvement se rapportant à la position du pied droit du conducteur pour ledit endroit prédéterminé, et en comparant ladite vitesse déterminée du véhicule au niveau dudit endroit prédéterminé par rapport aux données dans les données de définition de mouvement se rapportant à la vitesse du véhicule pour ledit endroit prédéterminé ; et
une étape d'émission permettant d'émettre un résultat d'évaluation par ledit dispositif évaluateur.
